# EUROPEAN PATENT APPLICATION

(11) **EP 4 527 927 A1**
(43) Date of publication of application: **26.03.2025**
(21) Application number: 23199032.6
(22) Date of filing: 22.09.2023
(51) Int. Cl.: C12N 15/113, A61P 11/00, C12N 15/10, C12N 15/867

(54) **OLIGONUCLEOTIDE-BASED STRATEGIES TO COMBAT RESPIRATORY VIRUSES**

(71) Applicant: Medizinische Hochschule Hannover, 30625 Hannover (DE); Fraunhofer-Gesellschaft zur Förderung der angewandten Forschung e.V., 80686 München (DE)
(72) Inventor: Schambach, Axel, 30625 Hannover (DE); Vollmer Barbosa, Philippe, 30167 Hannover (DE); Schwarzer, Adrian, 30625 Hannover (DE); Braun, Armin, 30916 Isernhagen (DE)
(74) Representative: Doll, Markus Alexander

(57) **Abstract**

The present invention relates to the field of oligonucleotide-based antiviral therapies. In particular, the invention discloses oligonucleotide molecules that may either be provided as RNA molecules capable of inducing RNAi-mediated immunity against human parainfluenza virus or as antisense oligonucleotides (ASOs), e.g., gapmers, targeting RNAs derived from human parainfluenza virus as well as oligonucleotide-based therapeutics utilizing said oligonucleotide molecules. In further aspects, the invention provides methods for screening and preparing an oligonucleotide-based therapeutic that can be used to treat an infection with a respiratory virus of interest. Such therapeutics can deliver the claimed oligonucleotides, e.g., via LNPs (lipid nanoparticles) or lipid anchors, or via lentiviral vectors or polymers such as, e.g., polyethylenimine (PEI vectors). Finally, the present invention provides a composition for use in treating a respiratory disease which comprises a lentiviral vector pseudotyped with a vesiculoviral envelope glycoprotein and at least one surface protein of influenza A virus and/or of respiratory syncytial virus.

## Description

The present invention relates to the field of oligonucleotide-based antiviral therapies. In particular, the invention discloses oligonucleotide molecules that may either be provided as RNA molecules capable of inducing RNAi-mediated immunity against human parainfluenza virus or as antisense oligonucleotides (ASOs), e.g., gapmers, targeting RNAs derived from human parainfluenza virus as well as oligonucleotide-based therapeutics utilizing said oligonucleotide molecules. In further aspects, the invention provides methods for screening and preparing an oligonucleotide-based therapeutic that can be used to treat an infection with a respiratory virus of interest. Such therapeutics can deliver the claimed oligonucleotides, e.g., via LNPs (lipid nanoparticles) or lipid anchors, or via lentiviral vectors or polymers such as, e.g., polyethylenimine (PEI vectors). Finally, the present invention provides a composition for use in treating a respiratory disease which comprises a lentiviral vector pseudotyped with a vesiculoviral envelope glycoprotein and at least one surface protein of influenza A virus and/or of respiratory syncytial virus.

Viral respiratory diseases are among the most common diseases in the world. Respiratory viruses are efficiently transmitted from person to person and affect all age groups. Many respiratory diseases occur due to infections with various cold viruses and are associated with comparably harmless disease progressions in most people. Nevertheless, even a simple cold often forces patients to stay in bed for several days, which in some cases can lead to considerable economic losses for employers.

In contrast, global pandemics resulting from infections with respiratory viruses have repeatedly led to high death rates in the past. For example, the influenza virus subtype A/H1N1, which became known as the "Spanish flu," is estimated to have claimed between 20 and 50 million lives worldwide in the early 20th century. As of September 2023, the COVID-19 pandemic, which halted public life all over the world, already claimed nearly 7 million lives (https://covid19.who.int/). In addition, the seasonal flu poses a high risk, especially for the elderly and/or immunocompromised. According to WHO estimates, flu epidemics lead to an estimated 3 to 5 million cases of severe illness and about 290,000 to 650,000 deaths worldwide each year (https://www.who.int/news-room/fact-sheets/detail/influenza-(seasonal)). Moreover, as climate change progresses, there is also growing concern that the incidence of viral zoonotic diseases, i.e., infectious diseases that are caused by viruses and can be transmitted from animals to humans, will increase in the future, making the occurrence of further pandemics more likely.

There is thus a growing need for effective therapies against respiratory virus infections.

One potential strategy to combat viral infections relies on the use of nucleic acid-based therapeutics that target viral RNAs via complementary Watson-Crick base pairing.

Some of these nucleic acid-based therapeutics are able to trigger the cleavage or degradation of their viral target RNAs, whereas others effectively block viral mRNA processing or translation. One group of nucleic acid-based therapeutics relies on a mechanism known as RNA-interference (RNAi). RNAi is an evolutionarily conserved defence mechanism which is used particularly by plants, fungi, nematodes and insects to fight viral infections. During RNAi-mediated antiviral immunity, short pieces of single stranded ribonucleic acid (known as siRNA) bind to virus-derived RNAs and, with the participation of several enzyme complexes, block the production of viral proteins. Since the RNAi machinery itself is also conserved in humans where it mainly serves to regulate our own gene expression, scientists have recognized the therapeutic potential of RNAi in combating viral infections in human patients for several years.

For instance, Bitko et al, 2004 discloses inhibition of respiratory viruses, including e.g. RSV and PIV, using nasally administered siRNA.

Bourdreau et al, 2009 describes a therapeutic RNAi approach that aims to reduce the expression of a gene. For this purpose, the efficacy and toxicity of shRNA and miRNA constructs were compared in vitro and in vivo. The resulting siRNA was identical in both cases. Vector-based delivery of the miRNA constructs was also investigated. Adeno-associated viruses (AAV) of serotype 1 were loaded with mRNA and shRNA constructs and introduced into cells.

WO 2006/062596 A2 describes the treatment of RSV and PIV using RNAi-inducing dsRNA and mentions other respiratory viral infections as possible targets. Intranasal and parenteral administration of the RNAi triggers is described as the route of administration. In contrast, delivery of the active compounds into the infected target cells by viral vector systems is not disclosed.

International patent application WO 2006/110688 A9 describes RNAi-inducing constructs (siRNA and shRNA) for the treatment of respiratory diseases and explicitly names the viruses HPIV1, HPIV2, HPIV3, HPIV4a and HPIV4b and as RNAi targets the nucleotide sequences of the (+)- and (-)-strand of the viral replication cycle. Over 8000 potentially applicable individual sequences are described. Also mentioned is the lentiviral insertion of the RNAi triggers in the embodiments. The use of a miRNA-embedded shRNA construct is, however, not described in this publication.

Antisense oligonucleotides (ASOs) form another group of nucleic acid-based therapeutics that are used in so-called antisense therapy. ASOs are short single stranded oligonucleotides typically made of DNA that are capable of binding to a complementary region within a target RNA to either initiate its enzymatic degradation or to sterically block the binding of cellular factors or machineries to the RNA.

The first ASO-based drug to ever receive FDA approval, Fomivirsen, has been developed for treating retinitis caused by cytomegalovirus (CMV) infection. However, in light of the COVID-19 pandemic, scientists also started to turn to the development of ASO-based therapeutics to combat infections with respiratory viruses. For instance, Zhu et al., 2022 found that intranasal administration of locked nucleic acid ASOs targeting the SARS-CoV-2 RNA in a COVID-19 mouse model potently suppressed viral replication in the lung of infected mice.

Given the existing threat of emerging respiratory viral variants, there is a need for new screening methods that can rapidly and reliably develop and test new and effective nucleic acid-based therapeutics.

This problem is solved by the present invention, in particular by the subject matter of the claims.

The present invention provides a screening method for an oligonucleotide-based therapeutic for treating an infection with a respiratory virus of interest, the method comprising:
a) Identifying at least one target sequence in an RNA derived from the respiratory virus of interest;
b) Generating one or more candidate oligonucleotide molecules comprising a nucleic acid sequence complementary to the at least one target sequence identified in step a), wherein the candidate oligonucleotide molecule is selected from the group consisting of an RNA molecule capable of inducing RNAi and an antisense oligonucleotide;
c) screening the candidate oligonucleotide molecules generated in step b) for their ability to induce a reduction of the titer of the respiratory virus of interest in lung tissue, wherein the screening comprises steps of
   i. providing cut slices obtained from lung tissue,
   ii. Introducing the one or more candidate oligonucleotide molecules generated in step b) into the cut slices,
   iii. Infecting the cut slices with the respiratory virus of interest,
   iv. Obtaining supernatant from the cut slices at a first time point and at least a second time point after infection;
   v. Determining the respiratory virus titre in the supernatants obtained at the first time point and at the at least second time point of step iv for each candidate oligonucleotide molecule,
wherein steps c) ii. and iii. may be performed in any order.

In the context of the present invention, the term "respiratory virus" refers to a virus capable of infecting the lung and causing respiratory tract infections. Respiratory viruses are considered to represent the most frequent causative agents of disease in humans (Boncristiani, 2009). The most common representatives of these viruses are well adapted for person-to-person transmission and circulate in a global scale (Boncristiani, 2009).

The respiratory virus may be any respiratory virus known to the skilled person, including, e.g., influenza virus, respiratory syncytial virus, parainfluenza virus, metapneumovirus, rhinovirus, coronavirus, adenovirus and bocavirus. The meaning of the term "respiratory virus" also extends to all mutant variants and serotypes of the above-mentioned viruses. For example, an influenza virus may be an influenza A virus, e.g., of strains H5N1 or H1N1. For example, a coronavirus may be a SARS virus, e.g., SARS-CoV2.

The meaning of the term "respiratory virus" may however also extend to viruses that are not primarily known as a causing agent for respiratory tract infections. For instance, Ebola virus has been found to attack lung tissue suggesting a pulmonary involvement in Ebola Virus Disease (Lalle et al., 2019). Likewise, Nipah virus has been shown to cause person-to-person transmittable respiratory infections and will thus be considered as a respiratory virus (Devnath and Masud, 2021). Accordingly, a respiratory virus in the context of the invention may also encompass Ebola virus and Nipah virus.

In the context of the invention, "treating an infection" is to be understood to comprise a curative medical therapy of a subject with the intent to cure, ameliorate or stabilize an infection. It is used synonymously with "treating a subject having an infection". The term "infection" refers to the invasion, growth and proliferation of germs, e.g., viruses, in the body of a subject, which may lead to disease. In some embodiments, treating an infection is intended to mean that the progression of the infection is to be slowed, stopped or, preferably, reversed to allow for a perceivable improvement of the infected subject's well-being and health. As used herein, the term "treating" may also encompass preventing, i.e., precluding, averting, obviating, forestalling, stopping, or hindering said infection from happening in the first place. Treating an infection may thus also explicitly include the prophylactic treatment of a subject, e.g., a subject known to be exposed to the virus or at risk of being exposed to the virus. Preferably, treatment is curative.

The subject of the herein disclosed invention can be a vertebrate, such as a mammal, a fish, a bird, a reptile, or an amphibian. Preferably, the subject of the herein disclosed methods is a mammal, e.g., a human, non-human primate, horse, pig, rabbit, dog, sheep, goat, cow, camel, cat, guinea pig, rat or mouse. In a particularly preferred embodiment, the subject is a human.

The term "oligonucleotide" is herein understood to refer to a short polymeric sequence of RNA or DNA nucleotides. Oligonucleotides may have a minimum length of 2 nucleotides (nt) and a maximum length of about 35 nt. However, typical oligonucleotides consist of about 12-25 nt. An oligonucleotide-based therapeutic is an agent suitable for pharmaceutical use that employs oligonucleotides capable of binding with high specificity to target RNAs of therapeutic interest via complementary Watson-Crick base pairing.

The candidate oligonucleotide molecules generated in step b) may be an RNA molecule capable of inducing RNAi. RNAi is a natural and conserved molecular mechanism for post-transcriptional regulation of gene expression. In brief, RNAi involves the processing of double-stranded (ds) RNAs into shorter double stranded fragments of usually 20 to 25 base pairs (bp) inside a cell. Afterwards, the antisense strand of this dsRNA duplex, which is also referred to as guide strand, is incorporated into a multi-protein complex known as RNA-induced silencing complex (RISC). After association, this complex is recruited to a target mRNA molecule of interest via complementary base-pairing between the guide strand and a target sequence within said mRNA molecule, thereby blocking the translation of the mRNA transcript into a polypeptide. In instances where the antisense strand binds to its target sequence with near perfect complementarity, RNAi promotes the cleavage and irreversible degradation of the target RNA. Thus, RNAi can modulate both the stability and translational efficacy of mRNAs.

The RNA molecules capable of inducing RNAi are referred herein also as "RNAi-triggers". RNAi triggers can be of natural origin due to transcription of endogenous genes and further processing of the resulting primary transcripts to yield the effective dsRNA duplexes. On the other hand, RNAi triggers can also be introduced artificially into cells via transfection of RNAi duplexes or via expression of RNAi precursors from viral vectors. Since RISC is specific only to RNAs and does not differentiate between human and viral RNA, RNAi is particularly suitable for developing therapies against infections by viruses that require RNA species to complete their replication cycle.

In another embodiment of the invention, the candidate oligonucleotide molecules generated in step b) may be antisense oligonucleotides (ASOs). ASOs are artificial single-stranded oligonucleotides of about 12-25 nt, more typically of 15-20 nt length that target RNAs via complementary base pairing. They are usually made of DNA that optionally comprises various chemical modifications to increase their efficacy, enzymatic stability and decrease their immunogenicity and off-target toxicity. A specific form of ASOs are so-called gapmers that a chimeric oligonucleotides formed of a central short region made of DNA nucleotides flanked by stretches of chemically modified ribonucleotides such as locked nucleic acids (LNAs).

ASOs exert their effects by two main mechanisms. On the one hand, ASOs can hybridize to their target RNAs to form DNA-RNA hybrids, which are recognized by RNase H, an endonuclease that triggers cleavage of the RNA strand of the DNA-RNA duplex. Gapmers in particular are specifically designed to catalyze RNase H-dependent degradation of complementary RNA targets. On the other hand, binding of ASOs to functional cis-acting elements within the target RNA may sterically block access of the cellular machinery to the RNA to, e.g., prevent translation or interfere with the splicing of the RNA.

The therapeutic use of ASOs is commonly referred to as antisense therapy.

Step a) of the method according to the invention requires the identification of sequences in RNA derived from a respiratory virus that are suitable for being targeted via RNAi or antisense therapy. In the context of the invention, an "RNA derived from a virus" typically is an mRNA transcribed from the genome of a respiratory virus. In case the respiratory virus does not comprise a genome made of DNA but rather of single-stranded (ss)- or double-stranded (ds) RNA, the RNA derived from a virus may also be the genome itself. RNA viruses use an enzyme known as RNA-dependent RNA polymerase (RdRp) for generating copies of their RNA genome. In case the respiratory virus comprises a negative-sense (-) RNA genome, RdRp is utilized by the virus to produce a positive-sense (+) RNA intermediate which can be translated into protein. Thus, in the context of the invention, the RNA derived from a virus can also be such a (+) RNA intermediate produced by negative-sense ssRNA viruses for gene expression.

Identification of suitable target sequences in an RNA derived from a virus is typically achieved in-silico, i.e. by using a suitable computer program for automated and preferably high-throughput alignment of nucleic acid sequences. The identification of suitable target sequences for RNAi or for antisense therapy can be carried out on the basis of empirically determined data, e.g., via various freely available online tools, wherein thermodynamic properties of the resulting RNAi or ASO molecule are usually taken into account. Sequence comparison between different virus strains and alignment of conserved regions is usually not automated. The skilled person is aware of programs and online tools capable of modelling RNA-RNA or DNA-RNA interactions to identify suitable sequences within viral RNA that can be targeted using RNAi or antisense therapy. For example, the freely accessible online tools splashRNA (Pelossof *et al.* 2017) or the miR30a design algorithm by Adams *et al.* 2017 can be used for this purpose.

Once a viral target RNA sequence is identified in step a), one or more candidate oligonucleotides molecules comprising a nucleic acid sequence complementary to said target sequence are designed using the aforementioned algorithms and generated in step b).

If the candidate oligonucleotide molecule is an RNA molecule capable of inducing RNAi, it may be, e.g. a small interfering RNA, a short hairpin RNA and/or artificial microRNA.

MicroRNAs (miRNAs) are single-stranded, non-coding RNAs that are transcribed from endogenous miRNA-genes in the genome. It is estimated that hundreds of distinct and evolutionary conserved miRNAs modulate the expression of more than 60% of all human protein-coding genes within virtually every biomolecular pathway (Catalanotto et al., 2016). Physiologically, miRNAs are generated from longer endogenous precursor transcripts (pri-miRNA), that form hairpin structures and are processed through two distinct endonucleolytic cleavage steps in the nucleus (via the enzyme Drosha) and cytoplasm (via the enzyme Dicer) into short RNA duplexes with a length of of 20-23, e.g., 22 base pairs (bp) and a dinucleotide 3' overhang at both ends. Based on its thermodynamic properties defined by its sequence composition, the antisense strand of these duplexes is incorporated into RISC to guide the complex to a complementary target RNA. Naturally occurring small interfering RNAs (siRNAs) rely on a similar effector pathway as miRNAs but originate from double-stranded RNA molecules rather than from hairpin structures. For instance, in many cases, siRNAs are naturally produced from double-stranded viral RNA. Mature siRNAs typically have a length of 20 to 25 nt and, similar to miRNAs, rely on RNAi to prevent target RNAs from being translated into protein. However, whereas naturally occurring miRNAs typically silence tens to hundreds of genes by binding to the 3'UTR regions of the corresponding mRNAs guided by their 8 base pair "seed region" followed by repression of translation, synthetic siRNAs typically exhibit a higher specificity, often binding to their target sequences with up to 100% sequence complementarity, inducing cleavage and degradation of their target mRNA via the RISC complex before translation. Shortly after the discovery of the RNAi pathway, scientist have recognized the therapeutic potential of synthetic siRNA-based drugs. The first siRNA therapeutic, Patisiran, was approved by the FDA in 2018 and many more are expected to follow. One challenge associated with siRNA-based drugs is site-specific delivery. This requires the use of suitable delivery systems as well as different chemical modifications that can stabilize the siRNA itself.

Short hairpin RNAs (shRNA) constitute an alternative to siRNA-based therapeutics. shRNAs are artificially designed single stranded RNA molecules that exhibit a characteristic hairpin turn. shRNAs typically have a length of 50-70 nt and form a stem-loop structure consisting of a 19 to 29 bp region of double-strand RNA (the stem) bridged by a region of predominantly single-stranded RNA (the loop) and a dinucleotide 3' overhang. Once they are introduced into a cell, they are processed similarly to miRNAs into short RNA duplexes that resemble siRNAs. One strand of this duplex is then incorporated into RISC to facilitate knock-down of a target gene. shRNAs can be encoded by an expression vector, such as a viral vector, which allows expression and processing of the shRNA construct inside a target cell. Optionally, they can also be produced by in vitro transcription of a linearized plasmid-based expression construct or via chemical oligonucleotide synthesis, e.g., by using nucleoside phosphoramidites as building blocks. Additionally, shRNAs might be expressed from expression plasmids, such as CpG-free expression vector plasmids.

shRNAs are normally transcribed by RNA polymerase III. However, the expression of exogenous shRNAs in cells can saturate the endogenous miRNA machinery, causing toxic effects to the cell. Moreover, imprecise processing of the stem loop structures of shRNAs may result in aberrant guide- and passenger-strand mediated off-target effects. To overcome these problems, shRNAs may be provided as a "shRNAmiRs", i.e., the shRNA is embedded within the backbone of a natural miRNA backbone. Such a shRNAmiR may also be referred to as an artificial miRNA or miRNA mimic. shRNAmiRs can be generated from Polymerase II promoters, enabling constitutive, tissue specific or inducible expression (Adams et al., 2017), as well as physiologic expression levels.

Therefore, the candidate oligonucleotide molecule produced in step b) of the present invention may also be an artificial miRNA or shRNAmiR. The skilled person is aware of suitable miRNA backbones that can be used for the provision of shRNAs to cells, which may include e.g. the backbone of miR30a, miR223, miR144 or miR33. The shRNAmiR constructs may be generated as described in the example below or in Adams et al., 2017, which is hereby incorporated by reference, and accordingly, may have a design that corresponds to the design of the shRNAmiR constructs disclosed in Adams et al., 2017. In brief, the shRNAmiR constructs preferably comprise flanking regions and, downstream of the stem loop of the miRNA, a binding sequence that is complementary to the RNA target sequence identified in step a) of the method according to the invention. A sequence complementary to said binding sequence may be arranged upstream of the stem loop of the miRNA, in order to allow formation of a hairpin structure. The stem loop sequence may be adjusted according to the respective miRNA backbone used. Polycistronic line up of different miRNA backbones in different combinations may be performed to achieve parallel knockdown of several targets. These may consist of different variations of miRNA backbones miR30a, miR223, miR33 and/or miR144 in double, triple or quadruple constructs (exemplified in Table 1). In between the backbones, spacer regions of 0-99 base pairs of noncoding RNA may be introduced into the lineup.

**Table 1: Exemplary combinations of different miRNA backbones that may be combined in a single shRNAmiR construct.**

| **5' miR backbone** | **Combination partner (in 5'-> 3' direction)** |
|---|---|
| miR30a | miR223; miR33; miR144; miR223_miR33; miR223_miR144; miR223_miR33_miR144; miR33_miR223; miR33_miR144; miR33_miR223_miR144; miR144_miR223; miR144_miR33; miR144_miR223_miR33 |
| miR223 | miR30a; miR33; miR144; miR30a_miR33; miR30a_miR144; miR30a_miR33_miR144; miR33_miR30a; miR33_miR144; miR33_miR30a_miR144; miR144_miR30a; miR144_miR33; miR144_miR30a_miR33 |
| miR33 | miR30a; miR223; miR144; miR30a_miR223; miR30a_miR144; miR30a_miR223_miR144; miR223_miR30a; miR223_miR144; miR223_miR30a_miR144; miR144_miR30a; miR144_miR223; miR144_miR30a_miR223 |
| miR144 | miR30a; miR223; miR33; miR30a_miR223; miR30a_miR33; miR30a_miR223_miR33; miR223_miR30a; miR223_miR33; miR223_miR30a_miR33; miR33_miR30a; miR33_miR223; miR33_miR30a_miR223 |

The candidate oligonucleotide molecule generated in step b) comprises a nucleic acid sequence that is complementary to a target sequence identified in step a) of the method according to the invention. In the context of the invention, this nucleic acid sequence is also referred to as "guide sequence". In case the candidate oligonucleotide molecule is an siRNA or an ASO, the candidate oligonucleotide molecule may also consist of such a guide sequence. In the context of the invention, a guide sequence is considered complementary to its target sequence when it has been designed to at least partially hybridize to said target sequence. Hybridization refers to a process wherein two single-stranded nucleic acid molecules anneal to each other via complementary Watson-Crick base pairing. In the case of DNA-RNA interactions, the nitrogenous base adenine present in the DNA strand thus pairs and forms hydrogen bonds with uracil in the opposing RNA strand strand, whereas adenine present in the RNA pairs with thymine in DNA. In the case of RNA-RNA interactions, the nitrogenous base adenine present in one RNA strand pairs and forms hydrogen bonds with uracil in the opposing strand. Regardless of whether the interactions occur between DNA and RNA or between two RNA molecules.cytosine pairs with guanine. Accordingly, the guide sequence is complementary to the target sequence if it comprises a sequence of complementary bases mirroring at least a part of the target sequence, such that it allows for RNAi or ASO-mediated RNA degradation/blockage.

In one embodiment, the candidate oligonucleotide molecule consists of or comprises a guide sequence that is 100% complementary to its target sequence, i.e., the guide sequence hybridizes over its entire length to the target RNA sequence, wherein each base of the guide sequence hybridizes to a corresponding complementary base of the target RNA sequence. In such a scenario, the candidate oligonucleotide molecule is highly specific to its target sequence and thus typically does not bind to other sequences within a given sample. If the candidate oligonucleotide candidate is an RNA capable of inducing RNAi, the guide sequence is preferably 20-23 nt, e.g., 22 nt long and fully complementary to the target sequence. If the candidate oligonucleotide ins an ASO, the guide sequence is preferably 12-16 nt and fully complementary to the target sequence.

However, in some embodiments, it may be preferable that at least 1, e.g., at least 2, at least 3, at least 4, or at least 5 bases of the guide sequence have mismatches with their target RNA sequence, i.e., the guide sequence does not bind with 100% complementarity to its corresponding target sequence. Accordingly, the guide sequence of the candidate oligonucleotide molecule may have a nucleic acid sequence that is merely 75-99%, e.g., 75%, 80%, 85%, 90%, 95% or 99% complementary to its target sequence. In such a scenario, the candidate oligonucleotide molecule still exhibits a very high specificity to its target sequence, while also allowing the recognition of variants thereof. This can be advantageous because of viral variability, particularly observed after mutations in the viral genome or for members of the same viral family.

In another embodiment, the guide sequence of the candidate oligonucleotide molecule hybridizes to the target RNA with only a relatively short stretch of its sequence. For example, naturally occurring miRNAs generally do not hybridize to a target RNA over their entire length. Rather, miRNAs only bind to their target mRNA with a short stretch of 6 to 8 nucleotides (nt) at their 5' end (the so-called seed sequence). Accordingly, the guide sequence of a candidate oligonucleotide molecule may already be considered complementary to a target sequence within the meaning of the present invention when it is complementary to at least a seed region of at least 5 to 15, e.g., 5, 6, 7, 8, 9, 10, 11, 12, 13, 14, or 15 nucleotides to its respective target.

The candidate oligonucleotide molecules can be generated by any suitable method. For example, the oligonucleotide molecules can be generated by chemical synthesis, which is typically done by commercial companies on a customized basis. Alternative methods for generating in particular RNA molecules capable of inducing RNAi are well known to the skilled person and include, e.g., in vitro-transcription or in vivo transcription from DNA templates introduced into cells.

In step c) of the method according to the invention, the candidate oligonucleotide molecules generated in step b) are screened for their ability to induce a reduction of the titer of the respiratory virus of interest in cut slices obtained from lung tissue.

Since the oligonucleotide-based therapeutic preferably is for use in a human subject, the lung tissue used for preparing the cut slices is preferably of human origin as well. The lung tissue should be viable, e.g., viable human lung tissue. The lung tissue thus preferably is human explant lung tissue that has been surgically removed from a human subject and is grown in an explant culture.

However, many respiratory viruses, including e.g., influenza A virus strains H5N1 or H1N1, are known to infect animals, in particular farm animals such as chickens or pigs. In some embodiments, the screening of the candidate oligonucleotide molecules may thus also be performed in lung tissues of animal origin, e.g., lung tissues from pigs, cattle, sheep, mice, rats, cotton rats, ferrets or chickens.

The preparation of the lung cut slices, referred herein also as precision-cut lung slices or PCLS, can be done as described in Danov et al., 2019, which is hereby incorporated by reference in its entirety. Briefly, human explant lung tissue is inflated by being filled with agarose. After polymerization of the agarose, the lung tissue is punched and cut with a tissue slicer to obtain 200 - 300 µm thick round slices with a diameter of about 8mm. The skilled person will be aware of alternative methods for producing lung cut slices of comparable thickness. Accordingly, the production of the lung cut slices is not necessarily limited to the method as described to Danov et al., 2019.

The freshly prepared lung cut slices are provided in a suitable culture medium, e.g., DMEM/F-12 supplemented with penicillin/streptomycin, and may be maintained under typical cell culture conditions. e.g., at a temperature of 37° at 5% CO₂ for 7-10 days.

In step ii) the candidate oligonucleotide molecules are introduced into the lung cut slices, which may be achieved by any suitable method known to the skilled person. For instance, the candidate oligonucleotide molecules may be introduced by means of transfection, e.g., by lipofection, calcium phosphate transfection or electroporation.

In other embodiments, the candidate oligonucleotide molecules may be introduced into the lung cut slices using a suitable vector. For instance, if the candidate oligonucleotide is one of the herein-described RNAi-trigger the vector may be an expression vector encoding said RNAi trigger. Examples of such expression vectors include but are not limited to minicircles, plasmids, cosmids, phages, viruses or artificial chromosomes. In some embodiments, the vector preferably is a viral vector, e.g., a lentiviral vector, an adenoviral vector or an adeno-associated viral vector. Expression vectors typically contain a cargo sequence comprising an expression cassette, i.e. the necessary elements that permit transcription of a nucleic acid into the candidate RNA molecule, such as a suitable promoter.

The ideal means for introducing the candidate oligonucleotide molecule into the lung cut slices largely depends on whether the candidate oligonucleotide molecules are provided as siRNAs and ASOs or shRNAs/shRNAmiRs. In case the candidate oligonucleotide molecules are siRNAs or ASOs, they usually do not require further endonucleolytic processing to exert their biological effect. Accordingly, they may be directly delivered into the lung cut slices via transfection, preferably via lipofection.

On the other hand, candidate oligonucleotide molecules that are shRNAs or shRNAmiRs are preferably introduced into the lung cut slices using a suitable expression vector, preferably a viral vector. They may however also be introduced by different means, e.g., via transfection, e.g., via lipofection of in vitro-transcribed shRNAmiR constructs.

After the introduction of the candidate oligonucleotide molecules, the lung cut slices are kept at a temperature of 32 to 40°C, e.g., 32, 33, 34, 35, 36, 37, 38, 39 or 40°C, preferably at 35°C before being infected with a suitable amount of the respiratory virus of interest, according to standard infection protocols. For instance, the lung cut slices may be infected with a virus titre of 2*10² -2*10⁶, e.g., of 2*10²- 2*10⁵, 2*10³-2*10⁶, 2*10²- 2*10⁴, 2*10³- 2*10⁵, 2*10⁴-2*10⁶ or of 2*10², 2*10³, 2*10⁴, 2*10⁵ or 2*10⁶ focus forming units (FFU) in 200µL. Infection of the lung cut slices with the respiratory virus of interest may take place, e.g., about 12-96 hours, e.g. about 12, 24, 36, 48, 60, 72, 84 or 96 hours after introduction of the candidate oligonucleotide molecules. Preferably, the lung cut slices are infected with the respiratory virus of interest 24-84 hours after introduction of the candidate oligonucleotide molecules. The time between the introduction of the candidate oligonucleotide molecules and viral infection may depend on the means for introducing the candidate oligonucleotide molecule into the lung cut slices. For instance, if the candidate oligonucleotide molecules, e.g. shRNAs/shRNAmiRs, are transduced with a viral vector into the lung cut slices, infection with the respiratory virus of interest may preferably be done about 72 hours afterwards to provide sufficient time for the expression and endonucleolytic processing of the candidate oligonucleotides molecules, e.g. shRNA/shRNAmiR, inside the cut slices. If, however, the candidate oligonucleotide molecules are transfected, e.g., as siRNAs or ASOs into the cell, infection with the respiratory virus of interest may be done earlier, e.g. already about 24 hours after introduction of the candidate oligonucleotide molecule. The skilled person will have no problem to determine the ideal time point for infecting the cell with the respiratory virus of interest within the limits of the present specification. In some embodiments, it may be even advantageous to introduce the candidate oligonucleotide candidates into the lung cut slices while simultaneously infecting them with the respiratory virus. In further embodiments, it is possible to first infect the lung cut slices with the respiratory virus of interest before introducing the candidate oligonucleotide molecule. For instance, the candidate oligonucleotide molecules can be introduced into the lung cut slices about 12-36 hours, e.g., 24 hours, after they were infected with the respiratory virus of interest.

After introducing the candidate oligonucleotide molecule and infecting the lung cut slices with the respiratory virus of interest, culture supernatant is obtained from the cultured cut slices at a first time point and at least a second time point.

The first time point should ideally be chosen shortly after infecting the lung cut slices with the respiratory virus of interest. For instance, the first time point may be 30 minutes to 2 hours after infection. Preferably, the supernatant is obtained from the cut slices at a first time point of about 1 hour after infection. In case the lung slices were first infected with the virus prior to introduction of the candidate oligonucleotide molecules, the first time point may be before or after introduction of the candidate oligonucleotide molecules, preferably, shortly before said introduction, e.g., about one hour before said introduction.

The second time point should ideally be chosen to be about 24-96 hours, e.g. about 12, 24, 36, 48, 60, 72, 84 or 96 hours after the first time point, and in any case, at least 6 hours after introduction of the candidate oligonucleotide molecules. Preferably, the second time point is about 48 to 72 hours, e.g., about 48, 60 or 72 hours after the first time point.

Afterwards, the respiratory virus titre in the supernatants obtained at the first time point and at the at least second time point is determined for each candidate oligonucleotide molecule to identify preferred candidates that most effectively reduced viral load between the two time points. In the context of the invention, viral load is considered to be "effectively reduced" in cases where the virus titre in the supernatant obtained at the second time point is reduced by at least 25%, preferably, by at least 35%, more preferably by at least 50% compared to the virus titre in the supernatant obtained at the first time point. In the most preferred embodiments, viral load is considered to be "effectively reduced", if the viral titre in the supernatant of the second time point is reduced by more than 50% compared to the viral titre in the supernatant of the first time point, e.g., by at least 60, 70, 80, 85, 90, 95 or 99%. In some embodiments, it is particularly preferred if the virus titre in the supernatant obtained at the second time point has been reduced by 100% compared to the virus titre of the first supernatant, i.e., if no traces of respiratory virus can be detected at all in the supernatant obtained at the second time point.

Determining the virus titre may be done by any suitable method known to the skilled person, e.g., it may be determined using a focus forming assay (FFA) in cell culture. In some embodiments, supernatants may also be collected from the cultured lung cut slices at additional time points, e.g., at a third, fourth or even fifth timepoint to monitor the effect of the candidate oligonucleotide molecules on viral titre over a longer period of time. The candidate oligonucleotide molecules that exhibited the most effective reduction in viral load during step c) of the method according to the invention are then preferably selected for producing the oligonucleotide-based therapeutic, which will be described in more detail below.

In preferred embodiments, the candidate oligonucleotide molecules generated in step b) of the method of the invention are further subjected to at least one additional step of validation prior to the screening step c). Hereby, the number of candidate oligonucleotide molecules screened in the lung cut slices in step c) of the method according to the invention can be already limited to a set of particular promising candidates.

In the context of the invention, this validation step is referred to as first validation step and may comprise:
i. Cloning an RNA sequence identified in step a) or parts thereof into a UTR of an mRNA molecule encoding a fluorescent protein to obtain a reporter mRNA;
ii. Introducing said reporter mRNA into a cell,
iii. Introducing the one or more candidate oligonucleotide molecules generated in step b) into the cell,
iv. Assessing the fluorescence intensity in the cells comprising the reporter mRNA to determine a knock-down efficiency for each candidate oligonucleotide molecule.

Fluorochrome-based knock-down (KD) reporter assays used for validating RNAi efficiency and potency of synthetic RNAi-triggers are known from the state in the art (e.g., Du et al., 2004; Kumar et al., 2003, Fellmann et al., 2011). Of note, these assays may also be used to assess the knock-down efficiency of ASOs. Briefly, an RNA target sequence identified in step a) of the method of the invention or a part thereof is placed into an untranslated region (UTR) of a typically constitutively expressed mRNA encoding a fluorescent protein, such as, e.g., BFP, GFP, mCherry or Venus. Because the target sequences of miRNAs are usually located within the 3'UTR of an mRNA, the target sequence identified in step a) is preferably introduced into the 3'UTR of the reporter mRNA as well.

The reporter mRNA is subsequently introduced in step ii) of the validation process into a cell in culture such as, e.g., a 32D cell or a 293T cell. For instance, the reporter mRNA may be an in vitro transcribed mRNA that is introduced into the cell by a suitable transfection method such as lipofection. Alternatively, a suitable expression vector, as described herein, may be used for introducing a transgene that expresses the reporter mRNA inside the cell under the control of a suitable promoter. Said promoter may be configured to facilitate constitutive transcription of the reporter mRNA within the cell, resulting in varying amounts of reporter transcripts depending on the strength of the promoter. This facilitates adjustable sensitivity towards the tested constructs. For instance, the promoter may be, e.g., an SFFV or an EFS promoter. The promoter may alternatively also be an inducible promoter, such as a tetracycline-inducible promoter that triggers transcription of the reporter mRNA only in the presence of an external stimulus, such as the addition of tetracycline or doxycycline. The skilled person will have no problems to select suitable expression vectors and promoters for expressing the reporter mRNA inside a cell. The vector may also be configured to integrate the transgene into the genome of the cell to obtain a reporter cell line that stably expresses the reporter mRNA.

For the purpose of the present invention, the above-described reporter mRNA assay is preferably further optimized as described in Adams et al., 2017. For this, the UTR, e.g. the 3'UTR of the reporter mRNA does not only comprise a single cognate RNA target sequence but rather a plurality or even all of the target sequences identified in step a) of the method according to the invention. As described in Adams et al. 2017, the plurality of RNA target sequences can be incorporated *en bloc* into the 3'UTR of the reporter mRNA, e.g., by using the gBlocks^{™}-technology provided by Integrated DNA Technologies (IDT). This way, the authors of Adams et al., 2017 managed to concatenate up to 65 shRNA target sequences in the 3' UTR of a BFP fluorescence reporter gene (designated as mTagBFP2). By including multiple target sites within a single UTR of a reporter mRNA, it is not necessary to generate a separate reporter mRNA construct and/or reporter cell line for each candidate oligonucleotide molecule. Rather, cells of a single reporter cell line comprising and/or expressing the herein described reporter mRNA may be used to validate a plurality or even all of the candidate oligonucleotide molecules generated in step b) of the method according to the invention. This allows for time-saving parallel candidate validation, as each candidate oligonucleotide molecule to be validated can be simply introduced into a separate cell of the same reporter cell line. Additionally, the reporter mRNA construct may be equipped with an antibiotic-resistance cassette such as, e.g., a puromycin resistance cassette, to enable positive selection of reporter-positive cells prior to the validation process. In case the reporter construct comprises a puromycin resistance cassete, selection may take place by supplementation with about 0.4 - 4 µg/mL puromycin which typically killes at least 90% of reporter-negative cells.

The candidate oligonucleotide molecule(s) generated in step b) of the method according to the invention is next introduced into the cell as already described herein, wherein each candidate oligonucleotide molecule is introduced into a separate cell comprising and/or expressing the reporter mRNA. Thus, if the candidate oligonucleotide molecule is an siRNA or ASO, it is preferably introduced via transfection, e.g., via lipofection. For instance, the siRNA/ASO may be introduced into the cell using lipid nanoparticles (LNPs). Further methods for introducing siRNA molecules or ASOs into a target cell are known to the skilled person. For example, siRNA molecules or ASOs may also be introduced using cationic polymers such as, e.g., polyethylenimine (PEI) or DEAE-dextran, or branched polymeric molecules known as dendrimers. The siRNA or ASO may alternatively also directly be labelled with a suitable lipophilic anchor, e.g., by directly conjugating the siRNA or ASO to lipid residues such as palmityl- or cholesterol-residues. In cases where the candidate oligonucleotide molecule is provided as an shRNA or an shRNAmiR, it is preferably introduced by means of a viral vector such as a lentiviral vector. Like siRNAs or ASOs, however, in vitro-transcribed shRNA or shRNAmiR may also be transfected into the the cells, e.g., via lipofection using, e.g., LNPs or via cationic polymers such as, e.g. PEI.

In the absence of an RNAi trigger/ASO or in case a candidate oligonucleotide molecule introduced in step iii) does not or only weakly bind to any of the RNA target sequences within the UTR of the reporter mRNA, the reporter mRNA is translated into the encoded fluorescent protein of choice. Accordingly, a corresponding fluorescent signal can be detected by methods known to those skilled in the art, e.g., via fluorescence-activated cell sorting (FACS), fluorescence microscopy or western blotting. If, however, a candidate oligonucleotide molecule that is to be validated comprises a guide sequence complementary to any of the target RNA sequences in the UTR of the reporter mRNA, it is expected to prevent its translation into a fluorescent protein by triggering its RNAi- or antisense-mediated knock-down. In consequence, the fluorescent signal produced by the reporter cell will be reduced or completely abolished (i.e. the signal will be below the detection limit of the method used for measuring the fluorescent signal). By assessing the fluorescence intensity in the cells comprising the reporter mRNA that have been exposed to a given candidate oligonucleotide molecule, the skilled person will be able to easily determine and calculate the knock-down efficiency for each candidate oligonucleotide molecule based on standard methods. This can be achieved, e.g., by comparing the effect of a given candidate oligonucleotide molecule on fluorescence intensity in the cells comprising the reporter mRNA to one or more reference or control shRNA/ASO constructs of known knock-down efficiency. Suitable reference shRNAs have been reported in the state of the art and include, e.g., shRNAs against Pten or Cebpa as described in Fellmann et al., 2011. Of course, to be used as controls, the UTR of the reporter mRNA must, in addition, also comprise the target sequence of said reference shRNAs.

Typically, fluorescent intensity in the cells comprising the reporter mRNA is assessed about 12-72 hours, preferably 24-60 hours, most preferably 48 hours after introduction of the candidate oligonucleotide molecules to provide sufficient time for reporter knock-down and degradation of sufficient amounts of residual fluorescent protein in the cytoplasm.

In general, a strong fluorescent signal and thus a high fluorescence intensity in relation to the one or more reference or control shRNAs/ASOs is associated with a weak knock-down efficiency of a candidate oligonucleotide molecule, whereas a weak fluorescent signal and thus a low fluorescent intensity in relation to the one or more reference or control shRNAs/ASOs is associated with a strong knock-down efficiency of a candidate oligonucleotide molecule.

At the end of the herein disclosed validation step, a candidate oligonucleotide molecule is considered to be validated and thus may be subjected to the screening step c) of the method of the invention when it exhibits a "strong" knock-down efficiency, i.e., an knock-down efficiency of about at least 70%, preferably about at least 80%, and most preferably at about at least 90% as determined by the fluorescence intensity in the cells comprising the reporter mRNA.

The method of the invention may alternatively or, preferably, in addition to the first validation step involving the reporter mRNA described herein, comprise another step of validation. In the context of the invention, this additional or alternative step of validation is herein referred to as second validation step and may comprise
I. Introducing the one or more candidate oligonucleotide molecules generated in step b) into a cell in cell culture,
II. Infecting the cell of (I) with the respiratory virus of interest,
III. Obtaining supernatant from the cell of (I) at a first time point and at least a second time point after infection;
IV. Determining the virus titre in the supernatants obtained at the first time point and at the at least second time point of step (III).

The second validation step resembles screening step c) of the method of the invention, with the main exception that the candidate oligonucleotide molecules generated in step b) are not screened for their ability to induce a reduction of the titer of the respiratory virus of interest in lung tissue but rather in "conventional" cell culture. Accordingly, the candidate oligonucleotide molecules and the respiratory virus of interest are introduced as described herein into a cell in culture rather than into a lung cut slice. The cell in cell culture may be any suitable cell line that is susceptible to infection by the respiratory virus of interest. In some embodiments, the cell may, e.g., be a LLCMK2 cell cultivated in a suitable cell culture medium such as, e.g., DMEM supplemented with 10% FCS + Penicillin/Streptomycin. Other cells that may be used in the second validation step include, e.g., Calu3 cells cultivated in a suitable cell culture medium such as, e.g., MEM supplemented with 10% FCS + Penicillin/Streptomycin.

As described herein, the candidate oligonucleotide molecule can be introduced into the cells by means of transfection, preferably by lipofection, in particular when the candidate oligonucleotide molecule is an siRNA or ASO, whereas, when the candidate oligonucleotide molecule is an shRNA/shRNAmiR, it is preferably introduced into the cell by means of a suitable expression vector such as a viral vector.

After introduction of the candidate oligonucleotide molecules, the cell is preferably incubated at a temperature of 32 to 40°C, e.g., of 32, 33, 34, 35, 36, 37, 38, 39 or 40°C, preferably at a temperature of 35°C.

The cell is next infected with a suitable amount of the respiratory virus of interest, according to standard infection protocols. For instance, the cell may be infected with a virus titre of 2*10²- 2*10⁶, e.g., of 2*10²- 2*10⁵, 2*10³- 2*10⁶, 2*10²- 2*10⁴, 2*10³- 2*10⁵, 2*10⁴- 2*10⁶ or of 2*10², 2*10³, 2*10⁴, 2*10⁵ or 2*10⁶ focus forming units (FFU) in 200µL. Infection of the cell with the respiratory virus of interest may take place about 12-96 hours , e.g. about 12, 24, 36, 48, 60, 72, 84 or 96 hours after introduction of the candidate oligonucleotide molecules. Preferably, the cell is infected with the respiratory virus of interest 24-84 hours after introduction of the candidate oligonucleotide molecules. The time between the introduction of the candidate oligonucleotide molecules and viral infection may depend on the means for introducing the candidate oligonucleotide molecule into the cell. If the candidate oligonucleotide molecules are transduced with a viral vector into the cell, infection with the respiratory virus of interest may preferably be done about 72 hours after introduction of the candidate oligonucleotide molecules to provide sufficient time for the expression and endonucleolytic processing of the candidate oligonucleotide molecules. If, in contrast, the candidate oligonucleotide molecules are transfected, e.g., as siRNAs or ASOs into the cell, infection with the respiratory virus of interest may already be done about 24 hours after introduction of the candidate oligonucleotide molecule. The skilled person will have no problem to determine the ideal time point for infecting the cell with the respiratory virus of interest within the limits of the present specification. In some embodiments, it may be even advantageous to introduce the candidate oligonucleotide into the cell and simultaneously infect the cell with the respiratory virus. Alternatively, the cell may first be infected with the respiratory virus of interest before introducing the candidate oligonucleotide molecule. For instance, the candidate oligonucleotide molecules may be introduced into the cell about 12-36 hours, preferably 24 hours, after it has been infected with the respiratory virus of interest.

After introducing the candidate oligonucleotide molecule and infecting the cell with the respiratory virus of interest, culture supernatant is obtained from the cultured cell at a first time point and at least a second time point and the virus titre in each supernatant is determined as previously described.

As the result of the second validation step, a candidate oligonucleotide molecule is considered to be validated and thus may be subjected to the screening step c) of the method of the invention when the viral titre in the supernatant of the second time point is reduced by more than 50%, preferably by more than 60%, 70% or 80% compared to the viral titre in the supernatant of the first time point.

In a preferred embodiment, the screening method according to the invention comprises both the first and the second validation step as disclosed herein prior to step c). However, the method may also comprise only one of the two validation steps, e.g., either only the first or only the second validation step, prior to step c). In a further embodiment, the screening method of the invention does not comprise any validation step after step b) and prior to step c).

The present invention further provides a method of preparing an oligonucleotide-based therapeutic for treating an infection of a respiratory virus of interest.

The method comprises steps of selecting a candidate oligonucleotide molecule capable of inducing-mediated immunity against a respiratory virus using the herein described screening method and providing a vector comprising or encoding said candidate oligonucleotide molecule to obtain the oligonucleotide -based therapeutic for treating a respiratory virus of interest.

As already described herein, the candidate oligonucleotide molecule selected by the screening method according to the invention can be provided as an siRNA, an shRNA,an shRNAmiR (also referred herein as artificial miRNA) or an ASO. The candidate oligonucleotide molecule is selected based on its efficiency to reduce viral load in lung cut slices during step c) of the screening method according to the invention.

In the context of the invention, the term "vector" refers to any means suitable for delivering an RNA molecule capable of inducing RNAi or an ASO to a target cell or tissue, in particular a cell of the respiratory tract, herein also referred to as airway cell. The choice of a suitable vector of the oligonucleotide-based therapeutic essentially depends on whether the candidate oligonucleotide molecule is to be provided in the form of an siRNA or ASO or in the form of an shRNA/shRNAmiR. In addition, the choice of vector depends on whether the candidate oligonucleotide molecule is to be expressed at its intended site of action or is provided as an in vitro-produced oligonucleotide molecule that needs to be transported to its target site. For instance, if the candidate oligonucleotide molecule is provided as an shRNA or an shRNAmiR, the vector preferably is an expression vector encoding the candidate oligonucleotide molecule, as described herein, i.e. it may be a minicircle, a plasmid, a cosmid, a phage, a virus or an artificial chromosome. Preferably, the expression vector is a viral vector, e.g., an adenoviral vector, an adeno-associated vector (AAV), or, most preferably a retroviral, e.g., lentiviral vector.

The expression vector comprises an expression cassette, i.e. the necessary elements that permit transcription of a cargo nucleic acid sequence into the candidate oligonucleotide molecule, e.g. the shRNA or shRNAmiR, such as a suitable promoter. In one embodiment, the expression vector may comprise a cargo sequence that encodes only a single candidate oligonucleotide molecule, e.g. a single shRNA or shRNAmiR, selected by the screening method of the invention. Alternatively, the expression vector may comprise a cargo sequence that encodes a plurality of different candidate oligonucleotide molecules, e.g., a plurality of different shRNAs or shRNAmiRs, selected by the screening method of the invention. This way, it is possible to express multiple different candidate oligonucleotide molecules with different target sequences from a single expression vector.

A person skilled in the art will be able to design suitable viral vectors to comprise a cargo DNA sequence encoding one or more candidate oligonucleotide molecules, e.g. one or more shRNAs or shRNAmiRs based on general knowledge and the prior art. For example, a stretch of cargo DNA, i.e., a transgene, encoding the one or more candidate oligonucleotide molecules may be operably linked to a suitable promoter and inserted into the viral vector of choice. The promoter used to express the one or more oligonucleotide molecules may be any constitutively active promoter known in the art, e.g., a Rous sarcoma virus (RSV) promoter, a human cytomegalovirus (CMV) promoter, an HIV promoter or a eukaryotic promoter such as e.g., EF1a, PGK1, UBC, or human beta actin. Alternatively, the promoter may be an inducible promoter that only drives expression of the one or more candidate oligonucleotide molecules in the presence or absence of a certain stimulus. For example, the inducible promoter may be a Tet-regulated promoter, i.e., it may comprise a tetracycline response element (TRE) that can be bound by a tetracycline transactivator (tTA) protein in the presence of tetracycline or an analogue thereof, e.g., doxycycline. The promoter may also be a cell-specific promoter which only drives expression in a particular type of cell, e.g., an airwaycell.

In a preferred embodiment, the vector furthermore is a cell-specific vector capable of targeting the one or more candidate oligonucleotide molecules, e.g. the one or more shRNAs or shRNAmiRs, to a cell or a tissue of interest, e.g., to cells of the respiratory tract. Accordingly, if the vector is a viral vector such as a lentiviral vector, the assembled viral vector is preferably pseudotyped, e.g., with one or more different viral envelope glycoproteins or a capsid to facilitate binding of the vector to the surface of the cell of interest, e.g., an airway cell. The term pseudotyping refers to the generation of viral vectors that carry foreign viral envelope glycoproteins on their surface or are encapsulated in a capsid of a different virus or viral serotype to specifically target only particular cell types of interest. The viral vector particles are usually assembled inside suitable packaging cells known in the art according to standard laboratory techniques before they are administered to the subject.

The expression vector used for delivering the candidate oligonucleotide molecule, e.g. the shRNA or shRNAmiR, may also be a non-pathogenic, genetically modified bacterium or yeast comprising a prokaryotic or eukaryotic vector. The vector comprises a DNA molecule encoding the candidate oligonucleotide molecule operably linked to a promoter that controls expression of said candidate oligonucleotide molecule, as described above. The non-pathogenic bacterium is preferably invasive, i.e., it is capable of entering a host cell and may be derived, e.g., from a non-pathogenic strain of *Escherichia coli, Listeria, Yersinia, Rickettsia, Shigella, Salmonella, Legionella, Chlamydia, Brucella, Neisseria, Burkolderia, Bordetella, Borrelia, Coxiella, Mycobacterium, Helicobacter, Staphylococcus, Streptococcus, Vibrio, Lactobacillus, Porphyromonas, Treponema,* or *Bifidobacteriae.* Preferably, the prokaryotic or eukaryotic vector is specifically designed for targeted delivery to a cell of interest, e.g., by expressing a ligand on its surface that can be recognized by a receptor on the target cell, e.g. an airway cell.

However, in some embodiments, it may also be advantageous not to express shRNAs/shRNAmiRs in situ from an expression vector. Rather, the candidate oligonucleotide molecule in the form of an shRNA/shRNAmiR may first be produced in vitro before being packaged into a suitable delivery vehicle for direct delivery to the target cell. The use of such transport vehicles as vectors is also preferred in cases where the candidate oligonucleotide molecule is not provided as shRNA or shRNAmiR, but as siRNA or ASO, since siRNAs or ASOs are typically not expressed in situ from an expression vector at the cell of interest, but are provided as constructs generated in vitro.

A person skilled in the art is aware of various types of vectors that may serve as delivery vehicles for in vitro generated oligonucleotide molecules such as ASOs, siRNAs or in vitro-transcribed shRNAs/shRNA/miRs and which are capable of selectively targeting the oligonucleotide to a cell of interest. In a preferred embodiment, the vector may e.g., comprise a lipid membrane that encapsulates the candidate oligonucleotide molecule. For example, the vector may be a liposomal or an exosomal carrier comprising the candidate oligonucleotide molecule as a cargo and, optionally, expressing a targeting moiety on its surface. In a particularly preferred embodiment, the vector is a lipid nanoparticle (LNP) which typically has a size of about 10 to 1000 nanometers. The LNP may optionally comprise various modifications, including variations of cholesterol, lipid-anchored polyethanilglycol, structural lipids and ionizable lipids. The vector may however also be a bacterial minicell. These types of carriers have the ability to entrap both hydrophilic therapeutic agents within their central aqueous core or lipophilic drugs within their bilayer compartment. The targeting moiety may be, e.g., a cell-specific antibody or ligand such as, e.g., a small peptide, polysaccharide or nucleic acid that can be bound by a receptor expressed on the surface of the cell of interest, e.g., a airway cell. Use of such a liposomal, exosomal or minicell vector has the benefit of allowing for fusion of the vector with the lipid membrane of the cell of interest e.g., during endocytosis to allow for the release of candidate oligonucleotide molecule into the cytoplasm of the cell.

In another embodiment, the vector may be a polymeric micro- or nanoparticle. The micro- or nanoparticle may be formed of any physiologically acceptable polymeric material known in the art, e.g., it may comprise a biopolymer such as polysaccharide selected from the group comprising cellulose, alginate or chitosan. The vector may also be a nanocarrier comprising, e.g., a hyperbranched dendritic polyglycerol polymer. Such a nanocarrier is an amphiphilic unimolecular nanocarrier of dendritic structure, and it comprises a dendritic core and at least one shell. Dendrimers may also be used. Further alternative vectors known to the skilled person include e.g., polyethylenimine (PEI)-based vectors.

In one embodiment, the vector releases the candidate oligonucleotide molecule at once, after the vector has reached its targeted destination inside the subject. In another possible embodiment, the vector may be configured in a fashion to allow for a sustained release of the candidate oligonucleotide molecule in a desired period of time. A person skilled in the art will be able to choose a suitable vector from the art depending on the type of oligonucleotide used, the route of administration, and the respective target cell to which the candidate oligonucleotide molecule is to be delivered. In addition, based on the known state of the art, the skilled person will be able to further modify the vector for optimized targeted delivery and release of the inhibitor.

In some embodiments, the in vitro-generated candidate oligonucleotide molecule may also not be incorporated into a delivery vehicle as described herein, but may alternatively be labelled with a suitable lipid anchor to improve the delivery of the oligonucleotide candidate molecule, e.g., by directly conjugating the in vitro produced ASO, siRNA or shRNA/shRNAmiR to lipid residues such as palmityl- (also referred to as 2'-O-hexadecyl or C16) or cholesterol-residues as described, e.g., in Raouane et al., 2012 or Brown et al., 2022. Accordingly, said lipid anchor may be considered herein to serve as a vector for delivery of the oligonucleotide candidate molecule.

The oligonucleotide-based therapeutic produced by the herein described method is preferably formulated in a pharmaceutical composition. Accordingly, oligonucleotide-based therapeutic can be provided in a formulation with one or more excipients, e.g. with a pharmaceutically acceptable carrier.

Using the herein described screening method of the present invention, the inventors were able to identify oligonucleotide molecules that are capable of inducing RNAi-mediated immunity against human parainfluenza virus or of inducing antisense-mediated degradation of RNAs derived from human parainfluenza virus. Human parainfluenza virus (HPIV) is a respiratory virus that can cause severe complications, including death, especially in immunosup-pressed patients (for example, stem cell transplant recipients or those with chronic lung disease). It is also the most common cause of pseudocroup in children. To date, there is neither a specific therapy nor an effective vaccine against HPIV.

HPIV is a single-stranded RNA virus of the Paramyxoviridae family with a negative sense (-) RNA genome encompassing about 15,000 nucleotides. HPIV has six essential genes designated as L, HN, M, PC, F, NP, which all constitute potential targets for RNAi mediating agents. As described in the example below, the inventors followed the steps of the herein described screening method to identify numerous novel target sequences within both the (-)RNA genome of HPIV and the (+)RNA intermediate formed for gene expression and subsequently generated a series of candidate oligonucleotide molecules expected to recognize said target sequences and thus to trigger RNAi or, in case of ASOs, to induce antisense-mediated degradation or blockage of the viral RNA.

Thus, the present invention further provides an oligonucleotide molecule capable of targeting an RNA derived from HPIV, wherein the oligonucleotide molecule is
a) an RNA molecule capable of inducing RNAi comprising a nucleic acid sequence having at least 90%, e.g., at least 95% or 100% sequence identity to SEQ ID NO: 1 to 56; or
b) an ASO comprising a nucleic acid sequence that corresponds to at least a subsequence of 10 or more nucleotides of any of SEQ ID NO: 1 to 56.

In the context of the invention, the term "RNA derived from HPIV" refers either to the negative-sense (-) RNA HPIV genome or the (+) RNA intermediate produced by HPIV for gene expression.

The term "targeting" is herein understood to mean that the oligonucleotide molecules of the invention are able to bind, i.e., to "hybridize" to a target RNA derived from HPIV via complementary Watson-Crick base pairing as defined herein.

SEQ ID NO: 1 to 56 relate to ribonucleic acid sequences. However, it is to be understood that a nucleic acid sequence that corresponds to at least SEQ ID NO: 1 to 56 or subsequences thereof may be a deoxyribonucleic acid sequence or ribonucleic acid sequence. It may even comprise both deoxyribonucleotides and ribonucleotides and/or modified nucleotides, e.g., in case the ASO is a chimeric gapmer, as described below.

In some embodiments, the oligonucleotide molecule of the invention is a synthetic siRNA. The synthetic siRNA is typically provided as an RNA duplex of 20 to 27 base pairs (bp) consisting of a sense and a complementary antisense strand. The antisense strand of the siRNA duplex comprises a nucleic acid sequence having at least 90%, preferably at least 95%, more preferably 100% sequence identity to any of SEQ ID NO: 1 to 56. Alternatively, the antisense strand of the siRNA duplex may also consist of a nucleic acid sequence having at least 90%, preferably at least 95%, more preferably 100% sequence identity to any of SEQ ID NO: 1 to 56. In other words, the siRNA antisense strand may comprise or consist of a nucleic acid sequence that comprises up to 2, i.e., 0, 1 or 2 nucleotide substitutions compared to any of SEQ ID NO: 1 to 56. Once the siRNA has been introduced into a cell, the antisense strand of the siRNA duplex serves as the guide strand, which is incorporated into RISC to target the complex to the HPIV-derived RNA for RNAi. In different embodiments, the oligonucleotide molecule may also be only one strand of an siRNA duplex, i.e. it may only be the antisense strand comprising or consisting of a nucleic acid sequence having at least 90%, preferably at least 95%, more preferably 100% sequence identity to any of SEQ ID NO: 1 to 56. In such a scenario, the RNA molecule capable of inducing RNAi-mediated immunity against HPIV may also consist of a nucleic acid sequence having at least 90%, preferably at least 95%, more preferably 100% sequence identity any of SEQ ID NO: 1 to 56.

In alternative embodiments, the RNA molecule capable of inducing RNAi may be an shRNA, whereas a nucleic acid sequence having at least 90%, preferably at least 95%, more preferably 100% sequence identity to any of SEQ ID NO: 1 to 56 forms a part of the stem of said shRNA. Accordingly, when the RNA molecule is an shRNA, it necessarily comprises additional nucleotides besides the nucleic acid sequence having at least 90%, preferably at least 95%, more preferably 100% sequence identity toany of SEQ ID NO: 1 to 56 to facilitate proper folding of the RNA molecule into the stem loop structure of an shRNA. Preferably, the shRNA is embedded into a miRNA backbone, also referred to as miRNA framework, as described herein, e.g., the framework of miR30a, miR223, miR144 or miR33. Accordingly, the RNA molecule may also be an shRNAmiR.

The skilled person will be aware that neither siRNAs nor miRNAs or shRNA/shRNamiR must be fully complementary to their target sequences to effectively suppress protein synthesis, i.e., he or she will know that some mismatches are admissible. Thus, if the herein disclosed siRNA antisense strand or the guide sequence of the shRNA/shRNamiR comprises one or two substitutions compared to any of SEQ ID NO: 1 to 56, it binds to its target sequence with imperfect complementarity but can nevertheless be a potent RNAi trigger that specifically and effectively induces cleavage of the target RNA or inhibits its translation into protein. However, in preferred embodiments, the nucleotides corresponding to positions 2-8 of SEQ ID NO: 1 to 56 are not substituted in the herein disclosed siRNAs or shRNAs/shRNAmiRs, as these positions form the so-called "seed" region critically for the specificity of an RNAi trigger. Moreover, multiple studies (e.g., Du et al., 2005; Ahmed and Raghava, 2011) suggest that mismatches affecting positions 4 -12 in siRNAs significantly reduce RNAi efficacy, whereas mismatches at positions 1, 2, 3, 18 and 19, in particular at positions 1 and 19, appear to affect RNAi efficacy only weakly or not at all (Ahmed and Raghava, 2011). In view of this, if the RNA molecule of the invention capable of inducing RNAi comprises or consists of a nucleic acid sequence having 90% or 95% sequence identity to any of SEQ ID NO: 1 to 56, it preferably does not comprise a substitution at a position that correspond to positions 2-12 of SEQ ID NO: 1 to 56. It may, e.g., comprise a substitution at a position that corresponds to position 1 and/or position 19 of SEQ ID NO: 1 to 56. An RNA molecule of the invention capable of inducing RNAi may also comprise or consist of a nucleic acid sequence that is shorter than the sequences of SEQ ID NO: 1 to 56, e.g., it may comprise or consist only of a subsequence spanning 20 or 21 nucleotides, e.g., the first 20 or 21 nt, of any of SEQ ID NO: 1 to 56.

The RNA molecule capable of inducing RNAi of the invention may comprise one or more chemical modifications that have a stabilizing effect, in particular, in case the RNA molecule is an siRNA. For instance, the RNA molecule may comprise a modification in its ribose sugar backbone, e.g., a 2'-O modification such as 2'-OMe, 2'-F, 2'-O-methoxyethyl (2'-MOE) or 2'-O-guanidinopropyl (2'-O-GP).

Additional modification that may be incorporated in the RNA molecule of the invention may include, e.g., 5-fluoro-2'-deoxyuridine and 2'-O-methyl phospshorodithioate moieties.

The aforementioned modifications as well as others that may be used in the herein disclosed RNA molecules are well known to the skilled person and are summarized e.g., in Selvam et al., 2017. Modifications may also be added to improve siRNA delivery as described above. For instance, one or more nucleotides in the passenger RNA strand of the siRNA duplex may be conjugated to a suitable lipophilic moiety or anchor such as, e.g., palmityl- (also referred to as 2'-O-hexadecyl or C16) or cholesterol-residues as described above. Palmityl modifications of siRNAs are known to the skilled person due to, e.g. Brown et al., 2022.

Using the method of the invention, the present inventors could show that at least twelve of the 56 RNA molecules disclosed herein were able to target an RNA derived from HPIV and induce a reduction of the HPIV titer in human PCLS when they were provided either as siRNAs or shRNA/shRNAmiR. The RNA molecules that most effectively induced RNAi-mediated immunity against HPIV comprised a nucleic acid sequence of any of SEQ ID NO. 1, 2, 3, 9, 12, 14, 15, 17, 18, 22, 23 and 29. Accordingly, in a preferred embodiment, the RNA molecule of the invention capable of inducing RNAi comprises a nucleic acid sequence of any of SEQ ID NO. 1, 2, 3, 9, 12, 14, 15, 17, 18, 22, 23 and 29. As described herein, the RNA molecule may also comprises a nucleic acid sequence comprising one or two substitutions compared to any of SEQ ID NO: 1, 2, 3, 9, 12, 14, 15, 17, 18, 22, 23 and 29. Accordingly, the RNA molecule capable of inducing RNAi may also comprise a nucleic acid sequence having at least 90%, or at least 95% sequence identity to any of SEQ ID NO: 1, 2, 3, 9, 12, 14, 15, 17, 18, 22, 23 and 29. If the RNA molecule capable of inducing RNAi is an siRNA, its guide strand may also consist a nucleic acid sequence of any of SEQ ID NO. 1, 2, 3, 9, 12, 14, 15, 17, 18, 22, 23 and 29 or a nucleic acid sequence having at least 90%, or at least 95% sequence identity thereto. Preferably, if the RNA molecule of the invention is an siRNA, it consists of a guide strand and, optionally, a complementary passenger strand according to table 7 of the example below. It thus may consist of a guide strand having a nucleic acid sequence of any of SEQ ID NO: 169, 171, 173, 175 or 177 and, optionally, a corresponding complementary passenger strand having a nucleic acid sequence of any of SEQ ID NO: 170, 172, 174, 176 or 178, respectively. The siRNA may alternatively also consist of a guide strand with a nucleic acid sequence having at least 90%, or at least 95% sequence identity to any of SEQ ID NO: 169, 171, 173, 175 or 177 and, optionally, a corresponding complementary passenger strand with a nucleic acid sequence having at least 90%, or at least 95% sequence identity to any of SEQ ID NO: 170, 172, 174, 176 or 178, respectively. In other words, the siRNA antisense or guide strand may consist of a nucleic acid sequence that comprises up to 2, i.e., 0, 1 or 2 nucleotide substitutions compared to any of SEQ ID NO: 169, 171, 173, 175 or 177, whereas, optionally, the corresponding complementary passenger strand may consist of a nucleic acid sequence that comprises up to 2, i.e., 0, 1 or 2 nucleotide substitutions compared to any of SEQ ID NO: 170, 172, 174, 176 or 178, respectively. However, the guide strand of the siRNA preferably does not comprise a substitution at a position that corresponds to positions 2-12 of any of SEQ ID NO: 169, 171, 173, 175 or 177. It may, e.g., comprise a substitution at a position that corresponds to position 1 and/or position 19 of SEQ ID NO: 169, 171, 173, 175 or 177. The guide strand of the siRNA of the invention may also consist of a nucleic acid sequence that is shorter than the sequences of SEQ ID NO: 169, 171, 173, 175 or 177, e.g., it may comprise or consist only of a subsequence spanning 25 or 26 nucleotides, e.g., the first 25 or 26 nt, of any of SEQ ID NO: : 169, 171, 173, 175 or 177.

In a particular preferred embodiment, the RNA molecule capable of inducing RNAi comprises a nucleic acid sequence of any of SEQ ID NO: 1, 18 and 23 or a nucleic acid sequence having at least 90%, or at least 95% sequence identity thereto, as these three candidates turned out to be particularly effective for inducing RNAi-mediated immunity against HPIV in human PCLS. SEQ ID NO: 1, 18 and 23 are all complementary to a target sequence in the (+)RNA intermediate produced by HPIV. As can be seen in the example below, RNA molecules comprising either SEQ ID NO: 1 or 23 effectively reduced HPIV titres in PCLS independent of whether they were provided as siRNAs or as shRNAmiRs with a miR-30a framework. By comparison, the RNA molecule comprising a nucleic acid of SEQ ID NO: 18 is preferably provided as an siRNA to effectively mediate RNAi-induced immunity against HPIV in lung tissue.

Therefore, if the RNA molecule is an siRNA, it preferably consists of a guide strand having a nucleic acid sequence of any of SEQ ID NO: 169, 173 or 175 or a nucleic acid sequence having at least 90%, or at least 95% sequence identity thereto and, optionally, a corresponding complementary passenger strand having a nucleic acid sequence of any of SEQ ID NO: 170, 174, or 176, respectively, or a nucleic acid having at least 90%, or at least 95% sequence identity thereto.

In another embodiment, the oligonucleotide molecule of the invention may be an ASO comprising a nucleic acid sequence that corresponds to at least a subsequence of 10 or more nucleotides of any of SEQ ID NO: 1 to 56. Said subsequence may correspond to any continuous stretch of at least 10 nt length present in any of SEQ ID NO: 1 to 56. For instance, the ASO may comprise a nucleic acid sequence that corresponds to at least the first or final 10 nucleotides of any of SEQ ID NO: 1 to 56, preferably, the final 10 nucleotides. The ASO may also comprise a nucleic acid sequence that corresponds to a subsequence of any of SEQ ID NO: 1 to 56 that is longer than 10 nt, e.g., it may comprise a nucleic acid sequence that corresponds to a continuous subsequence spanning 11, 12, 13, 14, 15, 16, 17, 18, 19, 20 or 21 nt of any of SEQ ID NO: 1 to 56. Preferably, the ASO comprises a nucleic acid sequence that corresponds to at least a subsequence of 12 or more nucleotides of any of SEQ ID NO: 1 to 56. In some embodiments, the ASO may also comprise a nucleic acid sequence that corresponds to the complete sequence of any of SEQ ID NO: 1 to 56 or consists thereof. Preferably, the ASO comprises a nucleic acid sequence that corresponds to at least a subsequence of 10 or more continuous nucleotides of any of SEQ ID NO: 1, 2, 3, 9, 12, 14, 15, 17, 18, 22, 23 and 29, more preferably of SEQ ID NO: 1, 18 and 23. Particular preferred ASOs of the invention comprise a nucleic acid sequence of any of SEQ ID NO: 204, 205 or 206.

The ASOs of the invention may be DNA ASOs.

The ASOs of the invention may comprise a variety of chemical modifications to improve their pharmacokinetic properties such as stability, specificity and membrane permeability, and minimize their cytotoxicity. For instance, the non-bridging oxygen atoms in the phosphate group of the nucleotides forming the ASO may be replaced by phosphorothioate (PS), resulting in the formation of PS bonds that are resistant to nuclease-based degradation. Moreover, the 2' position of the ribose may be modified with an alkyl moiety, such as a methyl (2'-OMe) or a methoxyethyl (2'-MOE) group. The ASO may also be a locked nucleic acid (LNA), a peptide nucleic acid (PNA) or a phosphorodiamidate morpholino oligomer (PMO). LNAs contain a constrained ribose ring having a O2'-C4'-methylene linkage. PNAs have a peptide-like N-(2-aminoethyl)glycine linkage to replace the ribose-phosphate DNA backbone. PMOs contain a backbone of morpholine rings connected by phosphorodiamidate linkages. Such modified ASOs are characterized by a high target affinity, improved pharmacokinetic profiles and nuclease resistance (Tarn et al, 2021).

In further embodiments, the herein disclosed ASOs may be gapmers, i.e., chimeric ASOs that consist of a central short region of deoxyribonucleotides flanked by a stretch of ribonucleotides in which the ribose ring is modified with 2'-OMe, 2'-MOE or LNA. Gapmers can induce RNase H-mediated cleavage of the target RNA with a relatively greater binding affinity and specificity than conventional ASOs (Tarn et al, 2021).

Additional chemical modifications that can be introduced into ASOs are described in the state of the art, e.g., in Tarn et al., 2021. Based on the herein disclosed information and the state of the art, the skilled person will have no difficulties in designing functional ASOs comprising the herein disclosed nucleic acid sequences or subsequences thereof.

As for siRNAs, the herein disclosed ASOs may further comprise modifications to improve ASO delivery. However, since a PS backbone assists the membrane translocation of ASOs, additional means of delivery may be dispensable for PS-ASOs. Nevertheless, the herein disclosed ASOs may as well be conjugated to suitable lipophilic moieties or anchors such as, e.g., palmityl- (also referred to as 2'-O-hexadecyl or C16) or cholesterol-residues as described above. ASOs may also be conjugated to cell-penetrating peptides (CPPs) for enhanced drug delivery, such as the polycationic HIV-1 Tat peptide, the hydrophobic residue-containing peptide and artificial poly-arginine peptides. ASOs made of PMO have also been conjugated in the past to a synthetic octa-guanidine dendrimer. Such conjugates are referred to as vivo-PMOs (Tarn et al., 2021).

The present invention further provides an oligonucleotide-based therapeutic for treating an infection of HPIV, wherein the therapeutic comprises one or more of the herein disclosed oligonucleotide molecules of the invention, or, in case the oligonucleotide is an shRNA/shRNamiR, a nucleic acid encoding them. Suitable delivery methods are known in the art or described herein. Preferably, the therapeutic comprises
a) a lentiviral vector comprising a nucleic acid encoding one or more of an RNA molecule comprising a sequence having at least 90% sequence identity to SEQ ID NO: 1 to 56,
b) a lipid nanoparticle vector comprising one or more of the herein disclosed oligonucleotide molecules,
c) a cationic polymer, selected from the group comprising a PEI vector, associated with one or more of the oligonucleotide molecules disclosed herein, or
d) one or more of the herein disclosed oligonucleotides linked to a lipophilic moiety.

The lentiviral vector of therapeutic a) may exhibit any of the features disclosed herein. As described herein, if the therapeutic comprises a lentiviral vector, the oligonucleotide molecule is preferably provided by the vector as an shRNA or an shRNAmiR, preferably as an shRNAmiR. Accordingly, the lentiviral vector comprises a cargo sequence encoding an shRNA or an shRNAmiR comprising a nucleic acid sequence having at least 90%, at least 95% or 100% sequence identity to any of SEQ ID NO: 1 to 56, preferably to any of SEQ ID NO: 1, 2, 3, 9, 12, 14, 15, 17, 18, 22, 23 or 29, and most preferably to any of SEQ ID NO: 1 or 23. The lentiviral vector may also comprise a cargo sequence encoding more than one candidate oligonucleotide molecule comprising a nucleic acid sequence having at least 90%, at least 95% or 100% sequence identity to any of SEQ ID NO: 1 to 56. For instance, it may comprise a cargo sequence that encodes a first shRNA or an shRNAmiR comprising SEQ ID NO. 1 and a second shRNA or an shRNAmiR comprising SEQ ID NO. 23.

Lipid nanoparticles for use as drug delivery vehicles for siRNAs were first approved in 2018. A lipid nanoparticle is typically spherical with an average diameter between 10 and 1000 nanometers. Solid lipid nanoparticles possess a solid lipid core matrix that can solubilize lipophilic molecules. The lipid core is typically stabilized by surfactants (emulsifiers). The emulsifier used depends on administration routes and is more limited for parenteral administrations. The lipids used for generating a lipid nanoparticle may include triglycerides (e.g. tristearin), diglycerides (e.g. glycerol bahenate), monoglycerides (e.g. glycerol monostearate), fatty acids (e.g. stearic acid), steroids (e.g. cholesterol), and waxes (e.g. cetyl palmitate) (https://en.wikipedia.org/wiki/Solid_lipid_nanoparticle). Typical next generation lipid nanoparticle compositions also comprise varying composition of structural lipids (e.g. MC3), cholesterol or beta-sitosterol, PEG (e.g., DSPG-PEG2000 or DMG-PEG2000) and ionisable or cationic lipids (e.g., DOPE or DOTAP).

The oligonucleotide molecules of the invention may also be delivered in association with cationic polymers such as, e.g., polyethylenimine (PEI) or DEAE-dextran, or branched polymeric molecules, e.g., hyperbranched polymers such as hyperbranched polyglycerol polymers or dendrimers. The association can be covalent or non-covalent, but typically is non-covalent. The cationic polymer, e.g., polyethyleneimine (PEI) is capable of complexing with nucleic acids via electrostatic interactions between its cationic groups and the negatively charged nucleic acids, thereby forming so called polyplexes. These polyplexes are characterized by high transfection efficiency due to the high buffering capacity of PEI, which facilitates endosomal escape of the gene payload (Schwarz and Merkel et al., 2017). The cationic polymers, e.g., the PEI vectors can be functionalized with a wide variety of polymers including oligosaccharides, polyethylene glycol) (PEG), poly(ε-caprolactone), and others. They may further carry, e.g., targeting moieties or comprise chemical modifications such as ester or disulfide bonds to yield degradable PEI analogs that exhibit reduced cytotoxicity.

As already described herein, the delivery of the oligonucleotide candidate molecule may also be improved by by directly linking the oligonucleotide molecule to lipophilic moieties. Exemplary lipophilic moieties for delivering RNAi triggers such as siRNAs or for delivery of ASOs include lipid residues such as palmityl- (also referred to as 2'-O-hexadecyl or C16) or cholesterol-residues as described, e.g., in Raouane et al., 2012 or Brown et al., 2022. Especially 2'-O-hexadecyl (C16) conjugates facilitate enhanced delivery and siRNA uptake into the alveolar and bronchiolar epithelium, which is why they may be particularly preferred for delivering the herein disclosed candidate oligonucleotide molecules to cells of the respiratory tract.

As described herein, when the vector is a lipid nanoparticle, a cationinc polymer such as, e.g., a PEI vector or a lipophilic moiety such as 2'-O-hexadecyl, the oligonucleotide molecule is preferably provided in the form of any of the herein disclosed siRNAs or ASOs. The oligonucleotide molecule may however also be provided as an in vitro produced shRNA or shRNAmiR. In some embodiments, the lipid nanoparticle or the cationic polymer, e.g., the PEI vector comprises only oligonucleotides molecules comprising or consisting of the same nucleic acid, e.g., the lipid nanoparticle comprises only RNA molecules comprising or consisting of a nucleic acid sequence of SEQ ID NO: 1 or ASOs comprising or consisting of SEQ ID NO: 204. Alternatively, the lipid nanoparticle or the cationic polymer, e.g., the PEI vector, may also comprise a mixture of oligonucleotide molecules comprising or consisting of different nucleic acid sequences, e.g. RNA molecules comprising or consisting of SEQ ID NO: 1 and RNA molecules comprising or consisting of SEQ ID NO: 18, RNA molecules comprising or consisting of SEQ ID NO: 1 and RNA molecules comprising or consisting of SEQ ID NO: 23 or RNA molecules comprising or consisting of SEQ ID NO: 18 and RNA molecules comprising or consisting of SEQ ID NO: 23. Alternatively, the lipid nanoparticle or the cationic polymer, e.g., the PEI vector, may also comprise a mixture of different ASOs disclosed herein, e.g., ASOs of SEQ ID NO: 204 and SEQ ID NO: 205, ASOs of SEQ ID NO: 205 and 206, or ASOs of SEQ ID NO: 204, 205 and 206. The lipid nanoparticle or the cationic polymer, e.g., the PEI vector, may also comprise a mixture of any of the herein disclosed RNAi triggers and ASOs, e.g., it may comprise RNA molecules comprising or consisting of SEQ ID NO: 1 and ASOs consisting of SEQ ID NO: 204. In such a scenario, the RNA molecules and ASOs present in the lipid nanoparticle or cationic polymer may bind to the same target sequence or to different ones.

The therapeutic may also comprise several lipid nanoparticles, cationic polymers such as PEI vectors or oligonucleotide-conjugates as described herein. In case the therapeutic comprises several lipid nanoparticles or cationic polymers such as PEI vectors, each lipid nanoparticle or cationic polymer comprises an oligonucleotide molecule comprising or consisting of a different nucleic acid sequence disclosed herein. For instance, the therapeutic may comprise a first lipid nanoparticle and/or cationic polymer, comprising an RNA molecule comprising or consisting of a nucleic acid sequence of SEQ ID NO: 1, a second lipid nanoparticle and/or cationic polymer comprising an RNA molecule comprising or consisting of a nucleic acid sequence of SEQ ID NO: 18, and, optionally, even a third lipid nanoparticle and/or cationic polymer comprising an RNA molecule comprising or consisting of a nucleic acid sequence of SEQ ID NO: 23. The therapeutic may also comprise, e.g., a first lipid nanoparticle and/or cationic polymer, comprising an ASO of SEQ ID NO: 204, a second lipid nanoparticle and/or cationic polymer comprising an ASO of SEQ ID NO: 205, and, optionally, even a third lipid nanoparticle and/or cationic polymer comprising an ASO of SEQ ID NO: 206. The therapeutic may also comprise several lipid nanoparticles or cationic polymers such as PEI vectors, wherein some lipid nanoparticle or cationic polymers within the therapeutic comprise any of the herein disclosed RNA molecules and some lipid nanoparticle or cationic polymers within the therapeutic comprise any of the herein disclosed ASOs. For instance, the therapeutic may comprise a first lipid nanoparticle and/or cationic polymer, comprising an RNA molecule comprising or consisting of a nucleic acid sequence of SEQ ID NO: 1, a second lipid nanoparticle and/or cationic polymer comprising an ASO of SEQ ID NO: 205. In such a scenario, the RNA molecules and ASOs used in the therapeutic may either bind to the same target sequence or to different ones, preferably to different ones. The therapeutic may also comprise several different oligonucleotide molecules of the invention linked to a liphophilic moiety such as 2'-O-hexadecyl (C16). For instance, the therapeutic may comprise a mixture of RNA molecules comprising or consisting of SEQ ID NO: 1 conjugated to 2'-O-hexadecyl, RNA molecules comprising or consisting of SEQ ID NO: 18 conjugated to 2'-O-hexadecyl, and/or RNA molecules comprising or consisting of SEQ ID NO: 23 conjugated to 2'-O-hexadecyl.

In yet another aspect, the present invention further provides a composition for use in treating a respiratory disease in a subject, wherein said composition is administered to the airways of the subject. The composition comprises a lentiviral vector pseudotyped with a vesiculoviral envelope glycoprotein and at least one surface protein of influenza A virus and/or at least one surface protein of respiratory syncytial virus.

In the context of the invention, a respiratory disease may be any pathological condition affecting the respiratory tract including the trachea, bronchi, bronchioles, alveoli, pleurae, pleural cavity, the nerves and muscles of respiration. Exemplary respiratory diseases may be the common cold, influenza, COVID-19 or pharyngitis, but may also include more severe diseases such as lung cancer.

The composition may be administered to the airways of a subject by any suitable means and by any suitable route known to the skilled person. For instance, the composition may be injected into the bloodstream of the subject. In a preferred embodiment, the composition however reaches the airways of the subject via inhalation, i.e., the composition may be provided to the patient as an aerosol. In the context oft the invention, the term "airways" refers to the organs of the respiratory tract of a subject. The airways can be subdivided into the upper airways and lower airways. The upper airways comprise the trachea as well as the main and lobar bronchi, whereas the lower airways comprise the smaller conducting bronchi and bronchioles.

A lentiviral vector is, as the name implies, derived from a genus of retroviruses known as lentivirus. Lentiviruses comprise e.g. human immunodeficiency virus (HIV), simian immunodeficiency virus (SIV), feline immunodeficiency virus (FIV) or equine infectious anemia virus (EIAV). Like that of other retroviruses, the genome of lentiviruses consists of a single-stranded (ss) positive sense RNA. During replication, the lentiviral ssRNA genome is converted into double-stranded (ds) DNA by a process known as reverse transcription. The reverse transcribed lentiviral dsDNA is subsequently integrated into the host cell's genome, which in turn replicates and transcribes the integrated lentiviral genes along with its own genes to produce new viral particles.

The lentiviral vector in the herein disclosed composition may thus be derived from a lentivirus selected from the group comprising HIV-1, HIV-2, SIV, FIV or EIAV. In a preferred embodiment, the lentiviral vector is derived from HIV-1.

The lentiviral, e.g., HIV-1, genome comprises three major structural genes: *gag, pol* and *env. gag* encodes the viral matrix (MA), capsid (CA) and nucleocapsid (NC), which collectively facilitate the assembly and release of the virus particles. The *pol* gene encodes the viral enzymes protease (PR), reverse transcriptase (RT) and integrase (IN), which govern viral replication. The HIV-1 *env* encodes the viral surface glycoprotein gp160, which is subsequently cleaved to form the surface protein gp120 and the transmembrane protein gp41 during viral maturation. In addition to the structural genes *gag, pol* and *env,* the HIV-1 genome furthermore comprises the two regulatory genes *tat* and *rev* as well as the four accessory genes *vif, vpr, vpu* and *nef*. Whereas *tat* encodes a transactivator required for viral transcription, *rev* encodes a protein that controls both splicing and export of viral transcripts. The four accessory genes are considered non-essential for viral replication, but are believed to increase its efficiency (German Advisory Committee Blood (Arbeitskreis Blut), Subgroup 'Assessment of Pathogens Transmissible by Blood', 2016, Human Immunodeficiency Virus (HIV). Transfus Med Hemother. 43(3), 203-222).

The lentiviral vector used in the herein disclosed composition preferably is a third-generation lentiviral vector.

In 1996, Naldini *et al.* reported the use of an HIV-based vector for *in vivo* gene delivery and stable transduction of non-dividing cells (Naldini *et al.,* 1996). Use of HIV-1-derived lentiviral vectors for *in vivo* gene transfer was however associated with considerable health risks. To meet these biosafety concerns, the first generation of lentiviral vector systems split the viral genome into three separate plasmids to avoid the formation of replication-competent viruses.

The first plasmid encoded the actual vector that was to be integrated into the host cell's genome. It comprised the transgene of interest functionally linked to a suitable promoter sequence as well as *cis*-elements necessary for polyadenylation, integration, initiation of reverse transcription and packaging (e.g., the long-terminal repeats, the packaging signal, the primer binding site, the polypurine tract or the Rev-responsive element). The second plasmid, known as packaging plasmid, comprised the genes encoding the viral proteins that contribute to packaging, reverse transcription and integration of the viral genome, i.e., *gag, pol,* the regulatory genes *tat* and *rev* as well as the four accessory genes *vif, vpu, vpr* and *nef.* The third plasmid (Env plasmid) expressed the viral glycoprotein for host cell receptor binding. Due to the physical separation of the packaging genes from the rest of the viral genome, this split genome design prevented viral replication after infection of the host cell.

To further improve the safety of lentiviral vectors, a second generation system was established by removing all accessory genes from the packaging plasmid, as they were found to constitute crucial virulence factors.

With the third generation of lentiviral vector systems, so-called self-inactivating (SIN) vectors were introduced. When lentiviral vectors are integrated into a host cell genome, the transgene cassette of the provirus comprising the gene of interest is flanked by two long terminal repeats (LTRs). The presence of these LTRs may promote the emergence of potentially harmful replication-competent recombinants. In addition, viral promoter/enhancer regions located in the LTRs could induce the expression of adjacent host genes, with potentially tumorigenic consequences. Moreover, the promoter/enhancer regions in these LTRs can transcriptionally interfere with the promoter driving the transgene as well as the neighboring genes. Therefore, promoter/enhancer sequences of the viral 3' LTR were removed by deleting a particular region inside the LTR (U3) from the DNA that was used to produce the viral RNA. Deletion of the U3 region effectively abolished the transcriptional activity of the LTR. Furthermore, *tat,* a regulatory gene driving viral transcription, was deleted from the packaging plasmid, whereas the second regulatory gene *rev* was provided from another separate, fourth, plasmid (Zufferey *et al.,* 1998; Schambach *et al.,* 2013). In case the 5' promoter driving the viral genomic mRNA is not HIV-derived and substituted by a CMV or RSV promoter, an extra tat plasmid may be omitted.

Accordingly, the lentiviral vector used in the herein disclosed composition is replication incompetent due to the split packaging design and self-inactivating (SIN) due to a deletion in the U3 region of the 3' LTR. It further lacks the genes *vif, vpr, vpu, nef,* and, optionally, *tat.*

Therefore, the lentiviral vector in the herein disclosed composition preferably comprises the following features:
a) a 5' LTR comprising a constitutively active heterologous promoter at the U3 position, a repeat region (R) and a U5 region,
b) a 5' UTR comprising a primer binding site (PBS), a splice donor site (SD), a packaging signal (ψ), a Rev-responsive element, and, optionally, a splice acceptor (SA) site,
c) an internal enhancer/promoter region operably linked to a cargo sequence,
d) RNA processing elements optionally comprising a Woodchuck hepatitis virus posttranscriptional regulatory element (PRE), and
e) a 3' LTR with a deleted (SIN) U3 region, a repeat region (R) and a U5 region.

The splice acceptor site is optional. The splice donor site is important, but vectors lacking it are functional. Presence of RNA processing elements such as a Woodchuck hepatitis virus posttranscriptional regulatory element (PRE) contribute to efficiency, and vectors without them are typically not generated in high titers. However, they can be concentrated.

Suitable 3^{rd} generation lentiviral vectors are e.g., known in the art and/or can be prepared by the skilled person. An exemplary lentiviral vector that can be used in the invention comprises a nucleic acid sequence of SEQ ID NO: 179. The lentiviral vector may also comprise a nucleic acid sequence having at least 80%, at least 85%, at least 90%, at least 95%, at least 96%, at least 97%, at least 98%, or at least 99% sequence identity to SEQ ID NO: 179. It may also consist of SEQ ID NO: 179. Alternatively, the lentiviral vector according to the invention may comprise a different promoter region than SEQ ID NO:179, e.g., another suitable promotor as taught herein.

Optionally, the viral enhancer/promoter regions located at the U3 position of the 5' LTR in the lentiviral vector may be replaced by a heterologous promoter to enhance expression of genomic RNA and to compensate for the loss of the lentiviral Tat protein. Therefore, the heterologous promoter preferably is a strong promoter, i.e., it drives constitutive expression of genes under its control. It may be selected from the group comprising a Rous sarcoma virus (RSV) promoter, a human cytomegalovirus (CMV) promoter, an HIV promoter (in case a lentiviral vector is used that is not derived from HIV-1 or HIV-2) or a eukaryotic promoter such as e.g., EF1a, PGK1, UBC, or human beta actin. It may also be an inducible promoter that only drives gene expression in the presence or absence of a certain stimulus, in particular, when expression of the vector would be toxic for the packaging cell. It may, e.g., be a Tet-regulated promoter, i.e., it may comprise a tetracycline response element (TRE) that can be bound by a tetracycline transactivator (tTA) protein in the presence of tetracycline or an analogue thereof, e.g. doxycycline. Preferably, the heterologous promoter is a constitutively active promoter derived from RSV or CMV.

The 5' UTR packaging signal (ψ) interacts with the nucleocapsid (NC) of the assembling Gag proteins to mediate packaging. Optimal packaging furthermore requires the trans-activation response element (TAR), U5, the primer binding site (PBS) and the group-specific antigen (Gag). The Rev protein additionally enhances the encapsidation of RNA containing the Rev-responsive element (RRE). The RRE also allows for higher expression of the viral genome and may suppress aberrant splice events (Schambach *et al.,* 2013). The 5' UTR may, optionally, further comprise multiple splice sites. In particular, the lentiviral vector may comprise a 5'UTR splice donor (SD) site and, optionally, a splice acceptor site (SA). In an optional embodiment, the 5' UTR may further comprise a polypurine tract (PPT) region, preferably downstream of the RRE. The PPT region is thought to increase the efficiency of reverse transcription and may even mediate nuclear entry of the lentiviral pre-integration complex, which forms after uncoating of the viral particle upon entry into the host cell (Schambach *et al.,* 2013).

The internal promoter/enhancer region is preferably a strong promoter, i.e., it drives constitutive expression of the cargo sequence under its control. It may be a promoter selected from the group comprising a viral promoter, a cellular promoter, a cell-specific promoter, an inducible promoter or a synthetic promoter.

It may, e.g., be a viral promoter selected from the group comprising a promoter derived from CMV, spleen focus-forming virus (SFFV), myeloproliferative sarcoma virus (MPSV), murine embryonal stem cell virus (MESV), murine leukemia virus (MLV) and simian virus 40 (SV40). Preferably, the internal promoter is a promoter derived from CMV or SFFV, most preferably from CMV. In another preferred embodiment, the promoter may also be a CAG promoter, i.e. a composite construct consisting of the CMV enhancer fused to the chicken beta-actin promoter and the rabbit beta-Globin splice acceptor site. The internal promoter/enhancer region may alternatively be a cellular promoter selected from the group comprising EF1a, EFS, PGK1 and UBC.

The internal promoter/enhancer may also be an inducible promoter, especially, if expression of the cargo sequence may lead to cytotoxicity. The promoter may thus be selected from the group comprising a TET-inducible promoter or a Cumate-inducible promoter.

The internal promoter/enhancer region may also be a hybrid or a synthetic promoter selected from the group comprising CAG and MND. A hybrid promoter is composed of several regulatory elements from different promoters/enhancers, which are joined to build a new promoter combining the desired features. In contrast, a synthetic promoter essentially consists of an array of transcription factor binding sites fused to a minimal promoter. A preferred hybrid promoter used for driving cargo sequence expression is CAG.

Finally, the internal enhancer/promoter region may comprise a chromatin opening element, i.e., a DNA sequence consisting of methylation-free CpG islands that either encompasses a housekeeping gene promoter or is operably linked to a heterologous promoter to confer reproducible, stable transgene expression.

The 3' UTR of the lentiviral vector according to the invention may comprise an RNA processing element, e.g., a Woodchuck hepatitis virus posttranscriptional regulatory element (PRE). The PRE may enhance cargo sequence expression by up to 10-fold and contributes to high virus titers (Schambach *et al.,* 2013).

To further prevent read-through transcription, the 3' end of the lentiviral vector may comprise upstream sequence elements (USE) that can be inserted into the 3' U3 region to improve 3' end processing and transcriptional termination. Preferably, the USE is a recombinant direct repeat of the USE derived from SV40 (2xSV USE). The 3' UTR may, optionally, further comprise target sites for differentiation-specific cellular miRNAs to enable post-transcriptional targeting of the vector mRNA.

Upon random or semi-random integration of a lentiviral vector into or next to a host cell's gene, the vector may act as a mutagen that dysregulates the gene's expression. In an optional embodiment, the space created by deletion of the U3 region in the 3'LTR of the lentiviral vector may therefore comprise an insulator sequence. Insulator sequences constitute barriers between regulator gene regions and thus may prevent vector-driven dysregulation of adjacent proto-oncogenes, i.e., cellular genes whose erroneous expression may provoke tumorigenesis (Schambach *et al.,* 2013).

To further increase biosafety of the composition according to the invention, the packaging plasmid encoding for Gag-Pol may be designed to comprise an inhibitory mutation in the *pol* region encoding for the integrase protein to render the lentiviral vector integration-deficient. Possible mutations may alter a triad of amino acid residues at the catalytic core of the integrase enzyme, e.g., D64, D116 and E152. Alternatively, amino acid changes may be introduced into the integrase DNA attachment site e.g. by mutating nucleotides within a 12 base-pair (bp) region of the vector 5' LTR U3 region or an 11 bp region of the 3'LTR U5 region (Shaw and Cornetta, 2014). Therefore, in an optional embodiment, the lentiviral vector according to the invention is a non-integrating lentiviral vector.

The lentiviral vector is preferably pseudotyped with a vesiculoviral envelope glycoprotein and at least one surface protein of influenza A virus and/or at least one surface protein of respiratory syncytial virus.

Vesiculovirus refers to a genus of negative-sense single-stranded RNA viruses in the family Rhabdoviridae, within the order Mononegavirales.

The vesicular envelope glycoprotein is selected from the group comprising MaraVg, VSVg, CoCVg and PyriVg.

Accordingly, in a first embodiment, the vesicular envelope glycoprotein used for pseudotyping the lentiviral vector is MaraVg and thus may have an amino acid sequence having at least 80%, preferably at least 85%, at least 90%, at least 95%, at least 96%, at least 97%, at least 98%, at least 99% or 100% sequence identity to SEQ ID NO: 180. Optionally, it may be encoded by a nucleic acid sequence having at least 80%, preferably at least 85%, at least 90%, at least 95%, at least 96%, at least 97%, at least 98%, at least 99% or 100% sequence identity to SEQ ID NO: 181. MaraVg is an envelope glycoprotein derived from Maraba virus. Maraba virus is a rhabdovirus best known for displaying broad tropism for both human and murine cancer cells.

In another embodiment, the vesicular envelope glycoprotein used for pseudotyping the lentiviral vector is VSVg, e.g., wild-type VSVg or a VSVg derivative capable of binding to the LDL-receptor or LDL-R family members. Preferably, the wild type VSVg is a glycoprotein derived from the Indiana VSV serotype and has an amino acid sequence having at least 80%, preferably at least 85%, at least 90%, at least 95%, at least 96%, at least 97%, at least 98%, at least 99% or 100% sequence identity to SEQ ID NO: 182. Optionally, it is encoded by a nucleic acid sequence having at least 80%, preferably at least 85%, at least 90%, at least 95%, at least 96%, at least 97%, at least 98%, at least 99% or 100% sequence identity to SEQ ID NO: 183. It may also be derived from the New Jersey VSV serotype and have an amino acid sequence having at least 80%, preferably at least 85%, at least 90%, at least 95%, at least 96%, at least 97%, at least 98%, at least 99% or 100% sequence identity to SEQ ID NO: 184. Optionally, it is encoded by a nucleic acid sequence having at least 80%, preferably at least 85%, at least 90%, at least 95%, at least 96%, at least 97%, at least 98%, at least 99% or 100% sequence identity to SEQ ID NO: 185. To achieve higher particle stability upon *in-vivo* administration and to evade potential recognition by the host's complement system, a thermostable and complement-resistant VSVg glycoprotein (VSVg ts) may alternatively be used, which may have an amino acid sequence having at least 80%, preferably at least 85%, at least 90%, at least 95%, at least 96%, at least 97%, at least 98%, at least 99% or 100% sequence identity to SEQ ID NO: 186. It may, optionally, be encoded by a nucleic acid sequence having at least 80%, preferably at least 85%, at least 90%, at least 95%, at least 96%, at least 97%, at least 98%, at least 99% or 100% sequence identity to SEQ ID NO: 187, and capable of binding to the LDL-R or LDL-R family members. VSVg confers a very broad tropism to viral vectors. Upon binding of VSVg to the LDL-receptor, the virion is taken up by the host cell via clathrin-mediated endocytosis. Besides providing high tropism, VSVg furthermore significantly increases the stability of the lentiviral vector, which allows for efficient concentration of viral titers by centrifugation, another essential feature for effective, virus-mediated gene transfer. Accordingly, pseudotyping of lentiviral vectors with VSV-G allows for robust transduction into a wide range of different cell types.

In a further embodiment, the vesicular envelope glycoprotein used for pseudotyping the lentiviral vector is CoCVg glycoprotein, i.e., a glycoprotein derived from Cocal virus. CoCVg is capable of binding to the LDL-receptor and it may, e.g., have an amino acid sequence having at least 80%, preferably at least 85%, at least 90%, at least 95%, at least 96%, at least 97%, at least 98%, at least 99% or 100% sequence identity to SEQ ID NO: 188. Optionally, CoCVg may be encoded by a nucleic acid sequence having at least 80%, preferably at least 85%, at least 90%, at least 95%, at least 96%, at least 97%, at least 98%, at least 99% or 100% sequence identity to SEQ ID NO: 189. In 2010, Trobridge *et al.* addressed some of the disadvantages of VSVg -pseudotyped lentiviral vectors, including the aforementioned cytotoxicity associated with constitutive VSVg expression, but also the inactivation of VSVg -pseudotyped vectors by human serum complement. The authors of this study demonstrated that lentiviral vectors pseudotyped with CoCVg glycoprotein derived from Cocal virus could be produced at titers as high as VSV-G-pseudotyped vectors, and exhibited both broad tropism and high stability. Importantly, pseudotyping with CoCVg also confers resistance to viral inactivation by human serum (Trobridge *et al.,* 2010).

In yet another embodiment, the vesicular envelope glycoprotein used for pseudotyping the lentiviral vector is PyriVg having an amino acid sequence having at least 80%, preferably at least 85%, at least 90%, at least 95%, at least 96%, at least 97%, at least 98%, at least 99% or 100% sequence identity to SEQ ID NO: 190. Optionally, it may be encoded by a nucleic acid sequence having at least 80%, preferably at least 85%, at least 90%, at least 95%, at least 96%, at least 97%, at least 98%, at least 99% or 100% sequence identity to SEQ ID NO: 191. The glycoprotein is thus capable of mediating entry into a host cell that can be entered by PyriVg having SEQ ID NO: 190. PyriVg is derived from the Piry virus, which, like VSVG, MARAV and COCV, belongs to the genus vesiculovirus. Similar to CoCVg, PyriVg was shown to be more resistant to complement-mediated inactivation by mammalian sera than VSVg (Tijani *et al.,* 2018). However, the cellular surface receptor facilitating the entry of Piry virus into a host cell is currently unknown.

The lentiviral vector may furthermore be pseudotyped with at least one surface protein of influenza A virus and/or at least one surface protein of respiratory syncytial virus.

The at least one surface protein of influenza A virus is selected from the group comprising surface proteins NA, HA and M2.

Influenza A hemagglutinin (HA) is a homotrimeric glycoprotein and serves as a viral attachment factor that can bind to sialic acid containing receptors at the surface of a target cell. Once the virus has bound to the cell, the target cell engulfs the virus via receptor-mediated endocytosis. Within the endosome, HA undergoes a series of conformational changes in response to a reduction in the pH, which results in a refolding of HA to expose a hydrophobic fusion peptide that is part of the N-terminus of one of its subunits. The exposed fusion peptide acts like a molecular grappling hook that inserts itself in the endosomal membrane. In consequence, the endosomal membrane and the viral membrane are brought into close contact to facilitate membrane fusion so that the viral RNA can enter the cell cytoplasm. The HA surface protein used for pseudotyping of the lentiviral vector may have an amino acid sequence having at least 80%, preferably at least 85%, at least 90%, at least 95%, at least 96%, at least 97%, at least 98%, at least 99% or 100% sequence identity to SEQ ID NO: 192. Optionally, HA may be encoded by a nucleic acid sequence having at least 80%, preferably at least 85%, at least 90%, at least 95%, at least 96%, at least 97%, at least 98%, at least 99% or 100% sequence identity to SEQ ID NO: 193.

Influenza A neuroaminidase (NA) is an enzyme that cleaves sialic acid and plays an important role during infection of a host cell by cleaving sialic acid groups to which HA becomes attached. NA furthermore facilitates the release of newly generated viral particles from the infected cell. The NA surface protein used for pseudotyping of the lentiviral vector may have an amino acid sequence having at least 80%, preferably at least 85%, at least 90%, at least 95%, at least 96%, at least 97%, at least 98%, at least 99% or 100% sequence identity to SEQ ID NO: 194. Optionally, NA may be encoded by a nucleic acid sequence having at least 80%, preferably at least 85%, at least 90%, at least 95%, at least 96%, at least 97%, at least 98%, at least 99% or 100% sequence identity to SEQ ID NO: 195.

Influenza A Matrix-2 (M2) protein is a proton selective viroporin capable of assembling into a homotetrameric proton channel. Once the virus is engulfed by its target host cell, M2 allowing passage of protons into the virion core in response to a reduction of the pH within the host endosome. The acidification of the interior of the virus facilitates the release of viral ribonucleoparticles into the host cell's cytoplasm upon membrane fusion. The M2 surface protein used for pseudotyping of the lentiviral vector may have an amino acid sequence having at least 80%, preferably at least 85%, at least 90%, at least 95%, at least 96%, at least 97%, at least 98%, at least 99% or 100% sequence identity to SEQ ID NO: 196. Optionally, M2 may be encoded by a nucleic acid sequence having at least 80%, preferably at least 85%, at least 90%, at least 95%, at least 96%, at least 97%, at least 98%, at least 99% or 100% sequence identity to SEQ ID NO: 197.

The at least one surface protein of respiratory syncytial virus (RSV) is selected from the group comprising surface proteins SH, F and G.

RSV small hydrophobic (SH) protein functions as a viroporin and can alter membrane permeabilty in the host. It was found to form oligomers that behave as cation-selective channels in the host cell membrane (Gan et al., 2012). The SH surface protein used for pseudotyping of the lentiviral vector may have an amino acid sequence having at least 80%, preferably at least 85%, at least 90%, at least 95%, at least 96%, at least 97%, at least 98%, at least 99% or 100% sequence identity to SEQ ID NO: 198. Optionally, SH may be encoded by a nucleic acid sequence having at least 80%, preferably at least 85%, at least 90%, at least 95%, at least 96%, at least 97%, at least 98%, at least 99% or 100% sequence identity to SEQ ID NO: 199.

The RSV Fusion (F) protein is embedded in the viral membrane, and, as the name implies, facilitates fusion with the membrane of a target host cell. The F surface protein used for pseudotyping of the lentiviral vector may have an amino acid sequence having at least 80%, preferably at least 85%, at least 90%, at least 95%, at least 96%, at least 97%, at least 98%, at least 99% or 100% sequence identity to SEQ ID NO: 200. Optionally, F may be encoded by a nucleic acid sequence having at least 80%, preferably at least 85%, at least 90%, at least 95%, at least 96%, at least 97%, at least 98%, at least 99% or 100% sequence identity to SEQ ID NO: 201.

The RSV G protein serves as an attachment glycoprotein of RSV. G is expressed on the surface of the virus and facilitates binding of RSV to the CX3CR1 chemokine receptor in human airway epithelial cells (Johnson et al., 2015). The G surface protein used for pseudotyping of the lentiviral vector may have an amino acid sequence having at least 80%, preferably at least 85%, at least 90%, at least 95%, at least 96%, at least 97%, at least 98%, at least 99% or 100% sequence identity to SEQ ID NO: 202. Optionally, G may be encoded by a nucleic acid sequence having at least 80%, preferably at least 85%, at least 90%, at least 95%, at least 96%, at least 97%, at least 98%, at least 99% or 100% sequence identity to SEQ ID NO: 203.

Accordingly, in one aspect, the composition for use according to the present invention comprises a lentiviral vector that is pseudotyped with VSVg and Influenza-NA.

In another aspect, the composition for use according to the present invention comprises a lentiviral vector that is pseudotyped with VSVg and Influenza-HA. In another aspect, the composition for use according to the present invention comprises a lentiviral vector that is pseudotyped with VSVg and Influenza-M2. In another aspect, the composition for use according to the present invention comprises a lentiviral vector that is pseudotyped with MaraVg and Influenza-NA. In another aspect, the composition for use according to the present invention comprises a lentiviral vector that is pseudotyped with MaraVg and Influenza-HA. In another aspect, the composition for use according to the present invention comprises a lentiviral vector that is pseudotyped with MaraVg and Influenza-M2. In another aspect, the composition for use according to the present invention comprises a lentiviral vector that is pseudotyped with CoCVg and Influenza-NA. In another aspect, the composition for use according to the present invention comprises a lentiviral vector that is pseudotyped with CoCVg and Influenza-HA. In another aspect, the composition for use according to the present invention comprises a lentiviral vector that is pseudotyped with CoCVg and Influenza-M2. In another aspect, the composition for use according to the present invention comprises a lentiviral vector that is pseudotyped with PyriVg and Influenza-NA. In another aspect, the composition for use according to the present invention comprises a lentiviral vector that is pseudotyped with PyriVg and Influenza-HA. In another aspect, the composition for use according to the present invention comprises a lentiviral vector that is pseudotyped with PyriVg and Influenza-M2. In another aspect, the composition for use according to the present invention comprises a lentiviral vector that is pseudotyped with VSVg and RSV-SH. In another aspect, the composition for use according to the present invention comprises a lentiviral vector that is pseudotyped with VSVg and RSV-F. In another aspect, the composition for use according to the present invention comprises a lentiviral vector that is pseudotyped with VSVg and RSV-G. In another aspect, the composition for use according to the present invention comprises a lentiviral vector that is pseudotyped with MaraVg and RSV-SH. In another aspect, the composition for use according to the present invention comprises a lentiviral vector that is pseudotyped with MaraVg and RSV-F. In another aspect, the composition for use according to the present invention comprises a lentiviral vector that is pseudotyped with MaraVg and RSV-G. In another aspect, the composition for use according to the present invention comprises a lentiviral vector that is pseudotyped with CoCVg and RSV-SH. In another aspect, the composition for use according to the present invention comprises a lentiviral vector that is pseudotyped with CoCVg and RSV-F. In another aspect, the composition for use according to the present invention comprises a lentiviral vector that is pseudotyped with CoCVg and RSV-G. In another aspect, the composition for use according to the present invention comprises a lentiviral vector that is pseudotyped with PyriVg and RSV-SH. In another aspect, the composition for use according to the present invention comprises a lentiviral vector that is pseudotyped with PyriVg and RSV-F. In another aspect, the composition for use according to the present invention comprises a lentiviral vector that is pseudotyped with PyriVg and RSV-G.

In further aspects, the lentiviral vector is pseudotyped with a vesiculoviral envelope glycoprotein, at least one surface protein of respiratory syncytial virus and at least one surface protein of influenza A virus.

For instance, the composition for use according to the present invention may comprise a lentiviral vector that is pseudotyped with VSVg, Influenza-NA and RSV-SH. In another aspect, the composition for use according to the present invention comprises a lentiviral vector that is pseudotyped with VSVg, Influenza-NA and RSV-F. In another aspect, the composition for use according to the present invention comprises a lentiviral vector that is pseudotyped with VSVg, Influenza-NA and RSV-G. In another aspect, the composition for use according to the present invention comprises a lentiviral vector that is pseudotyped with VSVg, Influenza-HA and RSV-SH. In another aspect, the composition for use according to the present invention comprises a lentiviral vector that is pseudotyped with VSVg, Influenza-HA and RSV-F. In another aspect, the composition for use according to the present invention comprises a lentiviral vector that is pseudotyped with VSVg, Influenza-HA and RSV-G. In another aspect, the composition for use according to the present invention comprises a lentiviral vector that is pseudotyped with VSVg, Influenza-M2 and RSV-SH. In another aspect, the composition for use according to the present invention comprises a lentiviral vector that is pseudotyped with VSVg, Influenza-M2 and RSV-F. In another aspect, the composition for use according to the present invention comprises a lentiviral vector that is pseudotyped with VSVg, Influenza-M2 and RSV-G. In another aspect, the composition for use according to the present invention comprises a lentiviral vector that is pseudotyped with MaraVg, Influenza-NA and RSV-SH. In another aspect, the composition for use according to the present invention comprises a lentiviral vector that is pseudotyped with MaraVg, Influenza-NA and RSV-F. In another aspect, the composition for use according to the present invention comprises a lentiviral vector that is pseudotyped with MaraVg, Influenza-NA and RSV-G. In another aspect, the composition for use according to the present invention comprises a lentiviral vector that is pseudotyped with MaraVg, Influenza-HA and RSV-SH. In another aspect, the composition for use according to the present invention comprises a lentiviral vector that is pseudotyped with MaraVg, Influenza-HA and RSV-F. In another aspect, the composition for use according to the present invention comprises a lentiviral vector that is pseudotyped with MaraVg, Influenza-HA and RSV-G. In another aspect, the composition for use according to the present invention comprises a lentiviral vector that is pseudotyped with MaraVg, Influenza-M2 and RSV-SH. In another aspect, the composition for use according to the present invention comprises a lentiviral vector that is pseudotyped with MaraVg, Influenza-M2 and RSV-F. In another aspect, the composition for use according to the present invention comprises a lentiviral vector that is pseudotyped with MaraVg, Influenza-M2 and RSV-G. In another aspect, the composition for use according to the present invention comprises a lentiviral vector that is pseudotyped with CoCVg, Influenza-NA and RSV-SH. In another aspect, the composition for use according to the present invention comprises a lentiviral vector that is pseudotyped with CoCVg, Influenza-NA and RSV-F. In another aspect, the composition for use according to the present invention comprises a lentiviral vector that is pseudotyped with CoCVg, Influenza-NA and RSV-G. In another aspect, the composition for use according to the present invention comprises a lentiviral vector that is pseudotyped with CoCVg, Influenza-HA and RSV-SH. In another aspect, the composition for use according to the present invention comprises a lentiviral vector that is pseudotyped with CoCVg, Influenza-HA and RSV-F. In another aspect, the composition for use according to the present invention comprises a lentiviral vector that is pseudotyped with CoCVg, Influenza-HA and RSV-G. In another aspect, the composition for use according to the present invention comprises a lentiviral vector that is pseudotyped with CoCVg, Influenza-M2 and RSV-SH. In another aspect, the composition for use according to the present invention comprises a lentiviral vector that is pseudotyped with CoCVg, Influenza-M2 and RSV-F. In another aspect, the composition for use according to the present invention comprises a lentiviral vector that is pseudotyped with CoCVg, Influenza-M2 and RSV-G. In another aspect, the composition for use according to the present invention comprises a lentiviral vector that is pseudotyped with PyriVg, Influenza-NA and RSV-SH. In another aspect, the composition for use according to the present invention comprises a lentiviral vector that is pseudotyped with PyriVg, Influenza-NA and RSV-F. In another aspect, the composition for use according to the present invention comprises a lentiviral vector that is pseudotyped with PyriVg, Influenza-NA and RSV-G. In another aspect, the composition for use according to the present invention comprises a lentiviral vector that is pseudotyped with PyriVg, Influenza-HA and RSV-SH. In another aspect, the composition for use according to the present invention comprises a lentiviral vector that is pseudotyped with PyriVg, Influenza-HA and RSV-F. In another aspect, the composition for use according to the present invention comprises a lentiviral vector that is pseudotyped with PyriVg, Influenza-HA and RSV-G. In another aspect, the composition for use according to the present invention comprises a lentiviral vector that is pseudotyped with PyriVg, Influenza-M2 and RSV-SH. In another aspect, the composition for use according to the present invention comprises a lentiviral vector that is pseudotyped with PyriVg, Influenza-M2 and RSV-F. In another aspect, the composition for use according to the present invention comprises a lentiviral vector that is pseudotyped with PyriVg, Influenza-M2 and RSV-G.

The inventors surprisingly found the combinations VSVg/Influenza-NA/RSV-SH and MaraVg/Influenza-NA/RSV-SH resulted in particular high transduction efficiencies in lung cut slices.

Accordingly, in a preferred embodiment, the at least one surface protein that is used for pseudotyping is Influenza-NA. In consequence, the composition for use of the present invention preferably comprises a lentiviral vector pseudotyped with a vesiculoviral envelope glycoprotein and at least Influenza NA.

Alternatively, the at least one surface protein that is used for pseudotyping is preferably RSV SH. In consequence, the composition for use of the present invention may comprise a lentiviral vector pseudotyped with a vesiculoviral envelope glycoprotein and at least RSV-SH.

In a more preferred embodiment, the composition for use of the present invention may comprise a lentiviral vector pseudotyped with a vesiculoviral envelope glycoprotein, Influenza-NA and RSV-SH.

The inventors showed particular advantages if the composition for use of the present invention comprises a lentiviral vector pseudotyped with VSVg, Influenza-NA and RSV-SH.

Alternatively, the composition for use of the present invention preferably comprises a lentiviral vector pseudotyped with MaraV-G, Influenza-NA and RSV-SH.

In further embodiments, the composition for use of the present invention comprises a lentiviral vector that is pseudotyped as described in the Table 2 below:

**Table 2: Combinations of different pseudotypes for construction of lentiviral vectors**

| **Vesiculoviral glykoprotein** | **Combination partner** |
|---|---|
| VSVg | Infl.-HA/Infl.-NA; lnfl.-HA/lnfl.-M2; Infl.-NA/Infl.-M2; Infl.-HA/Infl.-NA/lnfl.-M2; RSV-SH/RSV-G; RSV-SH/RSV-F; RSV-F/RSV-G; RSV-SH/RSV-G/RSV-F; Infl.-HA/RSV-SH/RSV-G; Infl.-HA/RSV-SH/RSV-F; Infl.-HA/RSV-F/RSV-G; Infl.-HA/RSV-SH/RSV-G/RSV-F; Infl.-NA/RSV-SH/RSV-G; Infl.-NA/RSV-SH/RSV-F; Infl.-NA/RSV-F/RSV-G; Infl.-NA/RSV-SH/RSV-G/RSV-F; Infl.-M2/RSV-SH/RSV-G; Infl.-M2/RSV-SH/RSV-F; Infl.-M2/RSV-F/RSV-G; Infl.-M2/RSV-SH/RSV-G/RSV-F; Infl.-HA/Infl.-NA/RSV-SH/RSV-G; Infl.-HA/Infl.-NA/RSV-SH/RSV-F; Infl.-HA/Infl.-NA/RSV-F/RSV-G; Infl.-HA/Infl.-NA/RSV-SH/RSV-G/RSV-F; Infl.-HA/Infl.-M2/RSV-SH/RSV-G; Infl.-HA/lnfl.-M2/RSV-SH/RSV-F; lnfl.-HA/lnfl.-M2/RSV-F/RSV-G; Infl.-HA/Infl.-M2/RSV-SH/RSV-G/RSV-F; Infl.-NA/Infl.-M2/RSV-SH/RSV-G; lnfl.-NA/lnfl.-M2/RSV-SH/RSV-F; Infl.-NA/Infl.-M2/RSV-F/RSV-G; Infl.-NA/Infl.-M2/RSV-SH/RSV-G/RSV-F; Infl.-HA/Infl.-NA/Infl.-M2/RSV-SH/RSV-G; Infl.-HA/Infl.-NA/Infl.-M2/RSV-SH/RSV-F; lnfl.-HA/lnfl.-NA/lnfl.-M2/RSV-F/RSV-G; Infl.-HA/Infl.-NA/Infl. -M2/RSV-SH/RSV-G/RSV-F |
| MaraVg | Infl.-HA/Infl.-NA; lnfl.-HA/lnfl.-M2; lnfl.-NA/lnfl.-M2; Infl.-HA/Infl.-NA/lnfl.-M2; RSV-SH/RSV-G; RSV-SH/RSV-F; RSV-F/RSV-G; RSV-SH/RSV-G/RSV-F; Infl.-HA/RSV-SH/RSV-G; Infl.-HA/RSV-SH/RSV-F; Infl.-HA/RSV-F/RSV-G; Infl.-HA/RSV-SH/RSV-G/RSV-F; Infl.-NA/RSV-SH/RSV-G; Infl.-NA/RSV-SH/RSV-F; Infl.-NA/RSV-F/RSV-G; Infl.-NA/RSV-SH/RSV-G/RSV-F; Infl.-M2/RSV-SH/RSV-G; Infl.-M2/RSV-SH/RSV-F; Infl.-M2/RSV-F/RSV-G; Infl.-M2/RSV-SH/RSV-G/RSV-F; Infl.-HA/Infl.-NA/RSV-SH/RSV-G; Infl.-HA/Infl.-NA/RSV-SH/RSV-F; Infl.-HA/Infl.-NA/RSV-F/RSV-G; Infl.-HA/Infl.-NA/RSV-SH/RSV-G/RSV-F; lnfl.-HA/lnfl.-M2/RSV-SH/RSV-G; Infl.-HA/lnfl.-M2/RSV-SH/RSV-F; lnfl.-HA/lnfl.-M2/RSV-F/RSV-G; Infl.-HA/Infl.-M2/RSV-SH/RSV-G/RSV-F; Infl.-NA/Infl.-M2/RSV-SH/RSV-G; lnfl.-NA/lnfl.-M2/RSV-SH/RSV-F; Infl.-NA/Infl.-M2/RSV-F/RSV-G; lnfl.-NA/lnfl.-M2/RSV-SH/RSV-G/RSV-F; Infl.-HA/Infl.-NA/Infl.-M2/RSV-SH/RSV-G; Infl.-HA/Infl.-NA/Infl.-M2/RSV-SH/RSV-F; lnfl.-HA/lnfl.-NA/lnfl.-M2/RSV-F/RSV-G; Infl.-HA/Infl.-NA/Infl.-M2/RSV-SH/RSV-G/RSV-F |
| CoCVg | Infl.-HA/Infl.-NA; lnfl.-HA/lnfl.-M2; lnfl.-NA/lnfl.-M2; Infl.-HA/Infl.-NA/lnfl.-M2; RSV-SH/RSV-G; RSV-SH/RSV-F; RSV-F/RSV-G; RSV-SH/RSV-G/RSV-F; Infl.-HA/RSV-SH/RSV-G; Infl.-HA/RSV-SH/RSV-F; Infl.-HA/RSV-F/RSV-G; Infl.-HA/RSV-SH/RSV-G/RSV-F; Infl.-NA/RSV-SH/RSV-G; Infl.-NA/RSV-SH/RSV-F; Infl.-NA/RSV-F/RSV-G; Infl.-NA/RSV-SH/RSV-G/RSV-F; Infl.-M2/RSV-SH/RSV-G; Infl.-M2/RSV-SH/RSV-F; Infl.-M2/RSV-F/RSV-G; Infl.-M2/RSV-SH/RSV-G/RSV-F; Infl.-HA/Infl.-NA/RSV-SH/RSV-G; Infl.-HA/Infl.-NA/RSV-SH/RSV-F; Infl.-HA/Infl.-NA/RSV-F/RSV-G; Infl.-HA/Infl.-NA/RSV-SH/RSV-G/RSV-F; lnfl.-HA/lnfl.-M2/RSV-SH/RSV-G; Infl.-HA/lnfl.-M2/RSV-SH/RSV-F; lnfl.-HA/lnfl.-M2/RSV-F/RSV-G; Infl.-HA/Infl.-M2/RSV-SH/RSV-G/RSV-F; Infl.-NA/Infl.-M2/RSV-SH/RSV-G; lnfl.-NA/lnfl.-M2/RSV-SH/RSV-F; Infl.-NA/Infl.-M2/RSV-F/RSV-G; Infl.-NA/Infl.-M2/RSV-SH/RSV-G/RSV-F; Infl.-HA/Infl.-NA/Infl.-M2/RSV-SH/RSV-G; Infl.-HA/Infl.-NA/Infl.-M2/RSV-SH/RSV-F; lnfl.-HA/lnfl.-NA/lnfl.-M2/RSV-F/RSV-G; Infl.-HA/Infl.-NA/Infl.-M2/RSV-SH/RSV-G/RSV-F |
| PyriVg | Infl.-HA/Infl.-NA; lnfl.-HA/lnfl.-M2; lnfl.-NA/lnfl.-M2; Infl.-HA/Infl.-NA/lnfl.-M2; RSV-SH/RSV-G; RSV-SH/RSV-F; RSV-F/RSV-G; RSV-SH/RSV-G/RSV-F; Infl.-HA/RSV-SH/RSV-G; Infl.-HA/RSV-SH/RSV-F; Infl.-HA/RSV-F/RSV-G; Infl.-HA/RSV-SH/RSV-G/RSV-F; Infl.-NA/RSV-SH/RSV-G; Infl.-NA/RSV-SH/RSV-F; Infl.-NA/RSV-F/RSV-G; Infl.-NA/RSV-SH/RSV-G/RSV-F; Infl.-M2/RSV-SH/RSV-G; Infl.-M2/RSV-SH/RSV-F; Infl.-M2/RSV-F/RSV-G; Infl.-M2/RSV-SH/RSV-G/RSV-F; Infl.-HA/Infl.-NA/RSV-SH/RSV-G; Infl.-HA/Infl.-NA/RSV-SH/RSV-F; Infl.-HA/Infl.-NA/RSV-F/RSV-G; Infl.-HA/Infl.-NA/RSV-SH/RSV-G/RSV-F; lnfl.-HA/lnfl.-M2/RSV-SH/RSV-G; Infl.-HA/lnfl.-M2/RSV-SH/RSV-F; lnfl.-HA/lnfl.-M2/RSV-F/RSV-G; Infl.-HA/Infl.-M2/RSV-SH/RSV-G/RSV-F; Infl.-NA/Infl.-M2/RSV-SH/RSV-G; lnfl.-NA/lnfl.-M2/RSV-SH/RSV-F; Infl.-NA/Infl.-M2/RSV-F/RSV-G; Infl.-NA/Infl.-M2/RSV-SH/RSV-G/RSV-F; Infl.-HA/Infl.-NA/Infl.-M2/RSV-SH/RSV-G; Infl.-HA/Infl.-NA/Infl.-M2/RSV-SH/RSV-F; lnfl.-HA/lnfl.-NA/lnfl.-M2/RSV-F/RSV-G; Infl.-HA/Infl.-NA/Infl.-M2/RSV-SH/RSV-G/RSV-F |

The lentiviral vector of the composition for use according to the invention furthermore comprises a cargo sequence, wherein expression of said cargo sequence in a cell of a lung may cure, ameliorate or stabilize a respiratory disease. The cargo sequence is operably linked to the internal promoter/enhancer region described in detail above. It comprises a nucleic acid selected from the group comprising a protein-coding gene, a miRNA, an shRNA or a IncRNA. In a particularly preferred embodiment, the lentiviral vector of the composition for use of the invention comprises a nucleic acid or cargo sequence which encodes one or more of the herein disclosed RNA molecules capable of inducing RNAi-mediated immunity against human parainfluenza virus, i.e. it encodes one or more of the herein disclosed RNA molecules comprising a nucleic acid sequence of any of SEQ ID NO: 1 to 56, preferably any of SEQ ID NO: 1, 2, 3, 9, 12, 14, 15, 17, 18, 22, 23 and 29 and most preferably any of SEQ ID NO: 1 or 23.

Throughout the invention, the term "about" is intended to be understood as "+/- 10%". If "about" relates to a range, it refers to both lower and upper limits of the range. "A" is intended to mean "one or more", if not explicitly mentioned otherwise.

All literature cited herein is herewith fully incorporated. The present invention is further illustrated, but not limited, by the following examples.

### Brief description of the Drawings

- **Fig. 1**: Knockdown efficiency of lentivirally encoded shmiR30a candidates (A, B) and of lentivirally encoded shmiR multiplex candidates (C, D, E) in an mTagBFP reporter assay.
- **Fig. 2**: Knockdown efficiency of siRNA candidates in an mTagBFP reporter assay.
- **Fig. 3**: Efficiency of lentivirally encoded shmiR30a candidates in an HPIV3 infection model in LLCMK2 cells.
- **Fig. 4**: Efficiency of siRNA candidates in an HPIV3 infection model in LLCMK2 cells pre and post infection.
- **Fig. 5**: Efficiency of lentivirally encoded shmiR30a candidates in an HPIV3 infection model in human PCLS.
- **Fig. 6**: Efficiency of transfected siRNA candidates in an HPIV3 infection model in human PCLS.
- **Fig. 7**: Efficiency of siRNA candidates in an HPIV3 infection model in LLCMK2 cells using lipofectamine, a LNP formulation or directly palmityl (P) - or cholersterol (C) - modified siRNA
- **Fig. 8**: Transduction efficiency of differentially pseudotyped lentiviral particles assessed by mCherry expression detected by fluorescent microscopy.
- **Fig. 9**: Schematic setup of plasmids used for vector production and screening assay of differentially pseudotyped lentiviral particles in Calu3 cells.
- **Fig. 10**: Transduction efficiency of VSVg/Influenza-NA/RSV-SH and MaraVg/Influenza-NA/RSV-SH pseudotyped lentiviral particles in PCLS.

### Example 1

### shmiR30a candidate generation

HPIV3 genome (strain C 243) negative and positive RNA strands were fed into the splashRNA tool (http://splashrna.mskcc.org/) and 30 candidates for the positive strand and 26 for the negative strand were generated (listed in table 3 and 4). For incorporation into the shmiR30a backbone, primer pairs including the predicted antisense guide sequence, the sense passenger sequence, the miR30 stem loop and 4 bp overhangs for BsmBl cloning were designed and ordered (Table 5 and 6). The miR30a-encoding lentiviral vector (pL40C.SFFV.mCherry.miR30N.Spacer.gb) was cut open with BsmBl restriction enzyme to release the spacer harboring the BsmBl recognition sites and leaving overhangs complementary to the designed primer pairs. Primer pairs were annealed and ligated into the shmiR30a backbone. Lentiviral vector production from the generated vectors was performed as described elsewhere (Daily, 2016, doi:10.1186/s12859-016-0930-z).

**Table 3: positive (+) strand target sequences for HPIV3**

| shRNA number | shRNA.name | Antisense.Guide.Sequence |
|---|---|---|
| 1 | LGene_195_v2 | SEQ ID NO: 1 |
| 2 | LGene_2829_v2 | SEQ ID NO: 2 |
| 3 | LGene_2473_v2 | SEQ ID NO: 3 |
| 4 | LGene_5792_v2 | SEQ ID NO: 4 |
| 5 | LGene_5977_v2 | SEQ ID NO: 5 |
| 6 | LGene_617_v2 | SEQ ID NO: 6 |
| 7 | LGene_2884_v2 | SEQ ID NO: 7 |
| 8 | LGene_325_v2 | SEQ ID NO: 8 |
| 9 | LGene_952_v2 | SEQ ID NO: 9 |
| 10 | LGene_2372_v2 | SEQ ID NO: 10 |
| 11 | LGene_641_v2 | SEQ ID NO: 11 |
| 12 | HNGene_1243_v2 | SEQ ID NO: 12 |
| 13 | HNGene_903_v2 | SEQ ID NO: 13 |
| 14 | HNGene_1316_v2 | SEQ ID NO: 14 |
| 15 | HNGene_248_v2 | SEQ ID NO: 15 |
| 16 | HNGene_1322_v2 | SEQ ID NO: 16 |
| 17 | FGene_1626_v2 | SEQ ID NO: 17 |
| 18 | FGene_1618_v2 | SEQ ID NO: 18 |
| 19 | FGene_1595_v2 | SEQ ID NO: 19 |
| 20 | FGene_1625_v2 | SEQ ID NO: 20 |
| 21 | FGene_746_v2 | SEQ ID NO: 21 |
| 22 | PCGene_1787_v2 | SEQ ID NO: 22 |
| 23 | PCGene_1268_v2 | SEQ ID NO: 23 |
| 24 | PCGene_1269_v2 | SEQ ID NO: 24 |
| 25 | PCGene_1594_v2 | SEQ ID NO: 25 |
| 26 | PCGene_1329_v2 | SEQ ID NO: 26 |
| 27 | MGene_364_v2 | SEQ ID NO: 27 |
| 28 | MGene_592_v2 | SEQ ID NO: 28 |
| 29 | MGene_550_v2 | SEQ ID NO: 29 |
| 30 | MGene_155_v2 | SEQ ID NO: 30 |

**Table 4: negative (-) strand target sequences for HPIV3**

| shRNA number | shRNA.name | Antisense.Guide.Sequence |
|---|---|---|
| 1 | HPIV3_LGene_Negative_1719_v2 | SEQ ID NO: 31 |
| 2 | HPIV3_LGene_Negative_869_v2 | SEQ ID NO: 32 |
| 3 | HPIV3_LGene_Negative_3739_v2 | SEQ ID NO: 33 |
| 4 | HPIV3_LGene_Negative_2177_v2 | SEQ ID NO: 34 |
| 5 | HPIV3_LGene_Negative_2631_v2 | SEQ ID NO: 35 |
| 6 | HPIV3_LGene_Negative_5788_v2 | SEQ ID NO: 36 |
| 7 | HPIV3_LGene_Negative_1512_v2 | SEQ ID NO: 37 |
| 8 | HPIV3_LGene_Negative_2632_v2 | SEQ ID NO: 38 |
| 9 | HPIV3_LGene_Negative_2203_v2 | SEQ ID NO: 39 |
| 10 | HPIV3_LGene_Negative_6386_v2 | SEQ ID NO: 40 |
| 11 | HPIV3_LGene_Negative_1768_v2 | SEQ ID NO: 41 |
| 12 | HPIV3_LGene_Negative_291_v2 | SEQ ID NO: 42 |
| 13 | HPIV3_LGene_Negative_2832_v2 | SEQ ID NO: 43 |
| 14 | HPIV3_HNGene_Negative_1440_v2 | SEQ ID NO: 44 |
| 15 | HPIV3_HNGene_Negative_1437_v2 | SEQ ID NO: 45 |
| 16 | HPIV3_HNGene_Negative_1568_v2 | SEQ ID NO: 46 |
| 17 | HPIV3_FGene_Negative_71_v2 | SEQ ID NO: 47 |
| 18 | H PIV3_FGene_Negative_299_v2 | SEQ ID NO: 48 |
| 19 | HPIV3_FGene_Negative_1377_v2 | SEQ ID NO: 49 |
| 20 | HPIV3_FGene_Negative_1101_v2 | SEQ ID NO: 50 |
| 21 | HPIV3_MGene_Negative_642_v2 | SEQ ID NO: 51 |
| 22 | HPIV3_PandCGene_Negative_1728_v2 | SEQ ID NO: 52 |
| 23 | HPIV3_PandCGene_Negative_1138_v2 | SEQ ID NO: 53 |
| 24 | HPIV3_PandCGene_Negative_1045_v2 | SEQ ID NO: 54 |
| 25 | HPIV3_PandCGene_Negative_807_v2 | SEQ ID NO: 55 |
| 26 | HPIV3_PandCGene_Negative_607_v2 | SEQ ID NO: 56 |

**Table 5: primer pairs for positive (+) strand target sequences for HPIV3**

| FW_Primer_Name | Primer. FW | REV_Primer_Name | Primer.REV |
|---|---|---|---|
| LGene_195_v2_FW | SEQ ID NO: 57 | LGene_195_v2_REV | SEQ ID NO: 58 |
| LGene_2829_v2_FW | SEQ ID NO: 59 | LGene_2829_v2_REV | SEQ ID NO: 60 |
| LGene_2473_v2_FW | SEQ ID NO: 61 | LGene_2473_v2_REV | SEQ ID NO: 62 |
| LGene_5792_v2_FW | SEQ ID NO: 63 | LGene_5792_v2_REV | SEQ ID NO: 64 |
| LGene_5977_v2_FW | SEQ ID NO: 65 | LGene_5977_v2_REV | SEQ ID NO: 66 |
| LGene_617_v2_FW | SEQ ID NO: 67 | LGene_617_v2_REV | SEQ ID NO: 68 |
| LGene_2884_v2_FW | SEQ ID NO: 69 | LGene_2884_v2_REV | SEQ ID NO: 70 |
| LGene_325_v2_FW | SEQ ID NO: 71 | LGene_325_v2_REV | SEQ ID NO: 72 |
| LGene_952_v2_FW | SEQ ID NO: 73 | LGene_952_v2_REV | SEQ ID NO: 74 |
| LGene_2372_v2_FW | SEQ ID NO: 75 | LGene_2372_v2_REV | SEQ ID NO: 76 |
| LGene_641_v2_FW | SEQ ID NO: 77 | LGene_641_v2_REV | SEQ ID NO: 78 |
| HNGene_1243_v2_F W | SEQ ID NO: 79 | HNGene_1243_v2_RE V | SEQ ID NO: 80 |
| HNGene_903_v2_FW | SEQ ID NO: 81 | HNGene_903_v2_REV | SEQ ID NO: 82 |
| HNGene_1316_v2_F W | SEQ ID NO: 83 | HNGene_1316_v2_RE V | SEQ ID NO: 84 |
| HNGene_248_v2_FW | SEQ ID NO: 85 | HNGene_248_v2_REV | SEQ ID NO: 86 |
| HNGene_1322_v2_F W | SEQ ID NO: 87 | HNGene_1322_v2_RE V | SEQ ID NO: 88 |
| FGene_1626_v2_FW | SEQ ID NO: 89 | FGene_1626_v2_REV | SEQ ID NO: 90 |
| FGene_1618_v2_FW | SEQ ID NO: 91 | FGene_1618_v2_REV | SEQ ID NO: 92 |
| FGene_1595_v2_FW | SEQ ID NO: 93 | FGene_1595_v2_REV | SEQ ID NO: 94 |
| FGene_1625_v2_FW | SEQ ID NO: 95 | FGene_1625_v2_REV | SEQ ID NO: 96 |
| FGene_746_v2_FW | SEQ ID NO: 97 | FGene_746_v2_REV | SEQ ID NO: 98 |
| PCGene_1787_v2_F W | SEQ ID NO: 99 | PCGene_1787_v2_RE V | SEQ ID NO: 100 |
| PCGene_1268_v2_F W | SEQ ID NO: 101 | PCGene_1268_v2_RE V | SEQ ID NO: 102 |
| PCGene_1269_v2_F W | SEQ ID NO: 103 | PCGene_1269_v2_RE V | SEQ ID NO: 104 |
| PCGene_1594_v2_F W | SEQ ID NO: 105 | PCGene_1594_v2_RE V | SEQ ID NO: 106 |
| PCGene_1329_v2_F W | SEQ ID NO: 107 | PCGene_1329_v2_RE V | SEQ ID NO: 108 |
| MGene_364_v2_FW | SEQ ID NO: 109 | MGene_364_v2_REV | SEQ ID NO: 110 |
| MGene_592_v2_FW | SEQ ID NO: 111 | MGene_592_v2_REV | SEQ ID NO: 112 |
| MGene_550_v2_FW | SEQ ID NO: 113 | MGene_550_v2_REV | SEQ ID NO: 114 |
| MGene_155_v2_FW | SEQ ID NO: 115 | MGene_155_v2_REV | SEQ ID NO: 116 |

**Table 6: Primer pairs for negative (-) strand target sequences for HPIV3**

| FW Primer Name | Primer.FW | REV Primer Name | Primer.REV |
|---|---|---|---|
| HPIV3_LGene_ Negative_1719_v2_FW | SEQ ID NO: 117 | HPIV3_LGene_ Negative_1719_v2_REV | SEQ ID NO: 118 |
| HPIV3_LGene_ Negative_869_v2_FW | SEQ ID NO: 119 | HPIV3_LGene_ Negative_869_v2_REV | SEQ ID NO: 120 |
| HPIV3_LGene_ Negative_3739_v2_FW | SEQ ID NO: 121 | HPIV3_LGene_ Negative_3739_v2_REV | SEQ ID NO: 122 |
| HPIV3_LGene_ Negative_2177_v2_FW | SEQ ID NO: 123 | HPIV3_LGene_ Negative_2177_v2_REV | SEQ ID NO: 124 |
| HPIV3_LGene_ Negative_2631_v2_FW | SEQ ID NO: 125 | HPIV3_LGene_ Negative_2631_v2_REV | SEQ ID NO: 126 |
| HPIV3_LGene_ Negative_5788_v2_FW | SEQ ID NO: 127 | HPIV3_LGene_ Negative_5788_v2_REV | SEQ ID NO: 128 |
| HPIV3_LGene_ Negative_1512_v2_FW | SEQ ID NO: 129 | HPIV3_LGene_ Negative_1512_v2_REV | SEQ ID NO: 130 |
| HPIV3_LGene_ Negative_2632_v2_FW | SEQ ID NO: 131 | HPIV3_LGene_ Negative_2633_v2_REV | SEQ ID NO: 132 |
| HPIV3_LGene_ | SEQ ID NO: 133 | HPIV3_LGene_ | SEQ ID NO: 134 |
| Negative_2203_v2_FW | | Negative_2203_v2_REV | |
| HPIV3_LGene_ Negative_6386_v2_FW | SEQ ID NO: 135 | HPIV3_LGene_ Negative_6386_v2_REV | SEQ ID NO: 136 |
| HPIV3_LGene_ Negative_1768_v2_FW | SEQ ID NO: 137 | HPIV3_LGene_ Negative_1768_v2_REV | SEQ ID NO: 138 |
| HPIV3_LGene_ Negative_291_v2_FW | SEQ ID NO: 139 | HPIV3_LGene_ Negative_291_v2_REV | SEQ ID NO: 140 |
| HPIV3_LGene_ Negative_2832_v2_FW | SEQ ID NO: 141 | HPIV3_LGene_ Negative_2832_v2_REV | SEQ ID NO: 142 |
| HPIV3_HNGene_ Negative_1440_v2_FW | SEQ ID NO: 143 | HPIV3_HNGene_ Negative_1440_v2_REV | SEQ ID NO: 144 |
| HPIV3_HNGene_ Negative_1437_v2_FW | SEQ ID NO: 145 | HPIV3_HNGene_ Negative_1437_v2_REV | SEQ ID NO: 146 |
| HPIV3_HNGene_ Negative_1568_v2_FW | SEQ ID NO: 147 | HPIV3_HNGene_ Negative_1568_v2_REV | SEQ ID NO: 148 |
| HPIV3_FGene_ Negative_71_v2_FW | SEQ ID NO: 149 | HPIV3_FGene_ Negative_71_v2_REV | SEQ ID NO: 150 |
| HPIV3_FGene_ Negative_299_v2_FW | SEQ ID NO: 151 | HPIV3_FGene_ Negative_299_v2_REV | SEQ ID NO: 152 |
| HPIV3_FGene_ Negative_1377_v2_FW | SEQ ID NO: 153 | HPIV3_FGene_ Negative_1377_v2_REV | SEQ ID NO: 154 |
| HPIV3_FGene_ Negative_1101_v2_FW | SEQ ID NO: 155 | HPIV3_FGene_ Negative_1101_v2_REV | SEQ ID NO: 156 |
| HPIV3_MGene_ Negative_642_v2_FW | SEQ ID NO: 157 | HPIV3_MGene_ Negative_642_v2_REV | SEQ ID NO: 158 |
| HPIV3_PandCGene_ Negative_1728_v2_FW | SEQ ID NO: 159 | HPIV3_PandCGene_ Negative_1728_v2_REV | SEQ ID NO: 160 |
| HPIV3_PandCGene_ Negative_1138_v2_FW | SEQ ID NO: 161 | HPIV3_PandCGene_ Negative_1138_v2_REV | SEQ ID NO: 162 |
| HPIV3_PandCGene_ Negative_1045_v2_FW | SEQ ID NO: 163 | HPIV3_PandCGene_ Negative_1045_v2_REV | SEQ ID NO: 164 |
| HPIV3_PandCGene_ Negative_807_v2_FW | SEQ ID NO: 165 | HPIV3_PandCGene_ Negative_807_v2_REV | SEQ ID NO: 166 |
| HPIV3_PandCGene_ Negative_607_v2_FW | SEQ ID NO: 167 | HPIV3_PandCGene_ Negative_607_v2_REV | SEQ ID NO: 168 |

### siRNA candidate generation

To compare shmiR30a to small interfering RNAs (siRNAs), target sequences of five well performing shmiR30a candidates were designed as analogous siRNAs (Table 7). For enhanced RISC loading, siRNA guide strand was 27 bp, passenger strand was 25 bp long.

**Table 7: positive (+) strand targeting siRNA sequences for HPIV3**

| siRNA name | AS guide strand | SS passenger strand |
|---|---|---|
| **1** (LGene_195_v2) | SEQ ID NO: 169 | SEQ ID NO: 170 |
| **12** (HNGene_1243_v2) | SEQ ID NO: 171 | SEQ ID NO: 172 |
| **18** (FGene_1618_v2) | SEQ ID NO: 173 | SEQ ID NO: 174 |
| **23** (PCGene_1268_v2) | SEQ ID NO: 175 | SEQ ID NO: 176 |
| **29** (MGene_550_v2) | SEQ ID NO: 177 | SEQ ID NO: 178 |

### Candidate screening in an mTagBFP reporter assay

For initial screening of RNAi candidates an mTagBFB reporter assay was performed as described earlier (Adams et al., 2017, 10.1016/j.biomaterials.2017.05.032). Briefly, 22 bp complementary sequences for all designed candidates were cloned *en bloc* into the UTR of a mTagBFP mRNA and introduced lentivirally into a 32D cell line. Upon successful knockdown by the candidates, the complete mRNA would be degraded which would be visualized by a loss of mTagBFP fluorescence.

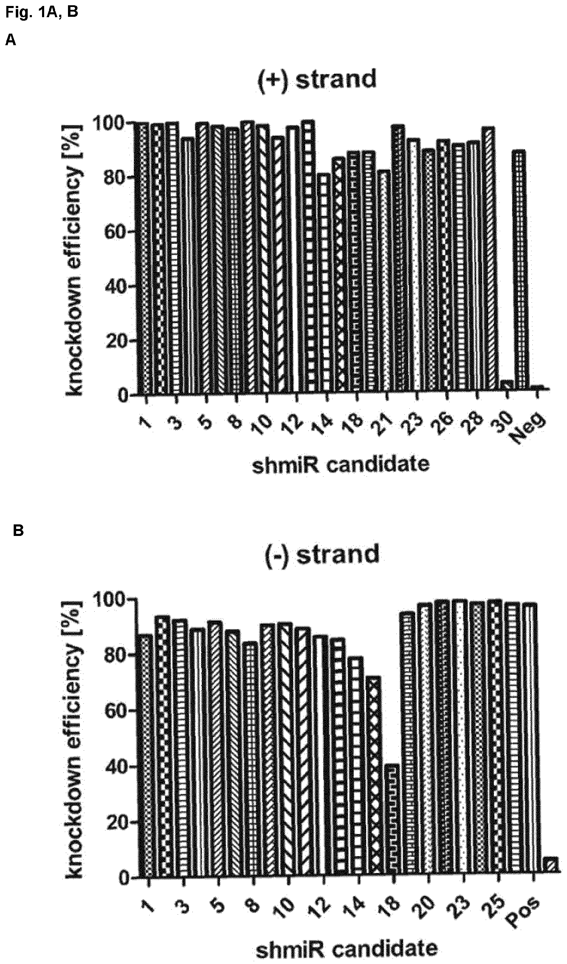

32D cells carrying the mTagBFP reporter were transduced with the shmiR30a candidates and cultured for 72 hours to enable reporter knockdown. Afterwards, mTagBFP fluorescence was assessed by flow cytometry and knockdown efficiency was calculated (results in Fig. 1A and B). The two most successful candidates of each gene were used for further testing.

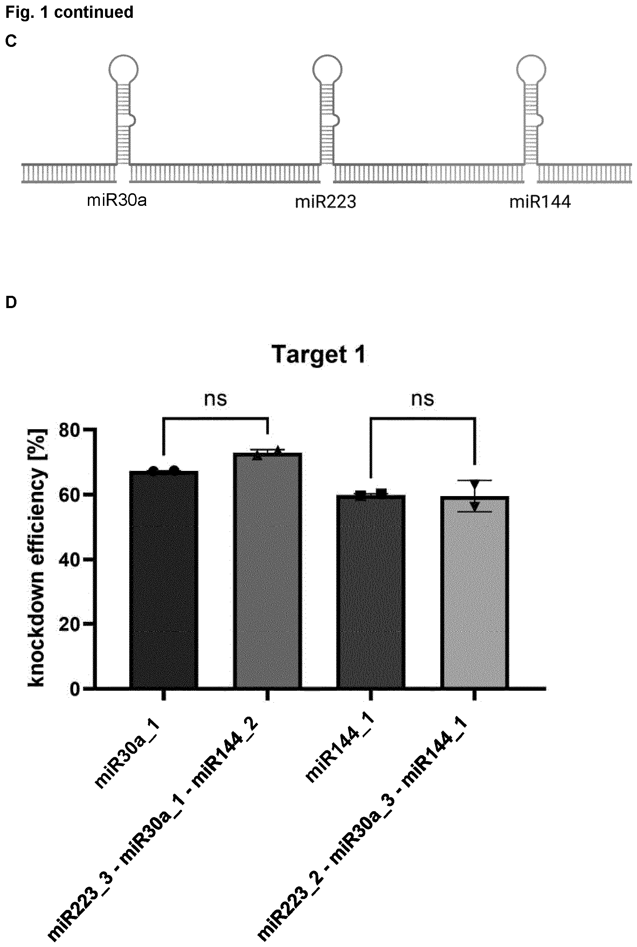

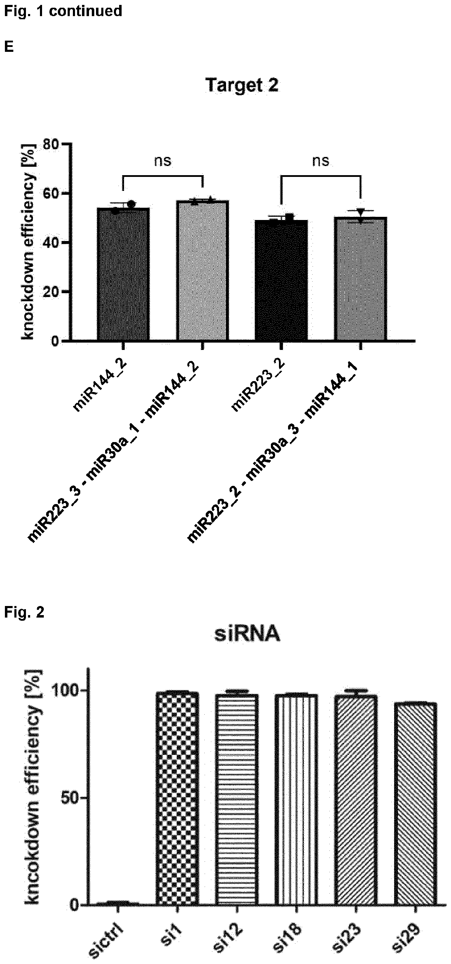

Polycistronic line up of different miRNA backbones (shmiR multiplexing) in different combinations may be performed to achieve parallel knockdown of several targets, as disclosed, e.g., in Liu et al., 2022. For shmiR multiplexing, miR30a, miR223 and miR144 backbones were cloned into in a single gene sequence at any given combination. In between the miRNA backbones, spacer regions of 0-99 base pairs of noncoding RNA may be introduced into the lineup (Fig. 1C). In a fluorescent reporter assay, the efficacy of the indicated lentivirally-encoded multiplex shmiR constructs was tested against their respective single shmiR constructs. Knockdown efficiency for two targets (Fig. 1D and E) was assessed three days after transduction with the shmiRs. Multiplex shmiRs performed equally efficient than the respective single targeting shmiRs.

For assessment of siRNA candidates, 293T cells carrying the mTagBFP reporter were transfected with the siRNA candidates using Lipofectamine RNAiMax and cultured for 48 hours to enable reporter knockdown. Afterwards, mTagBFP fluorescence was assessed by flow cytometry and knockdown efficiency was calculated (results in Fig. 2).

### shmiR30a candidate screening in an HPIV3 infection model in LLCMK2

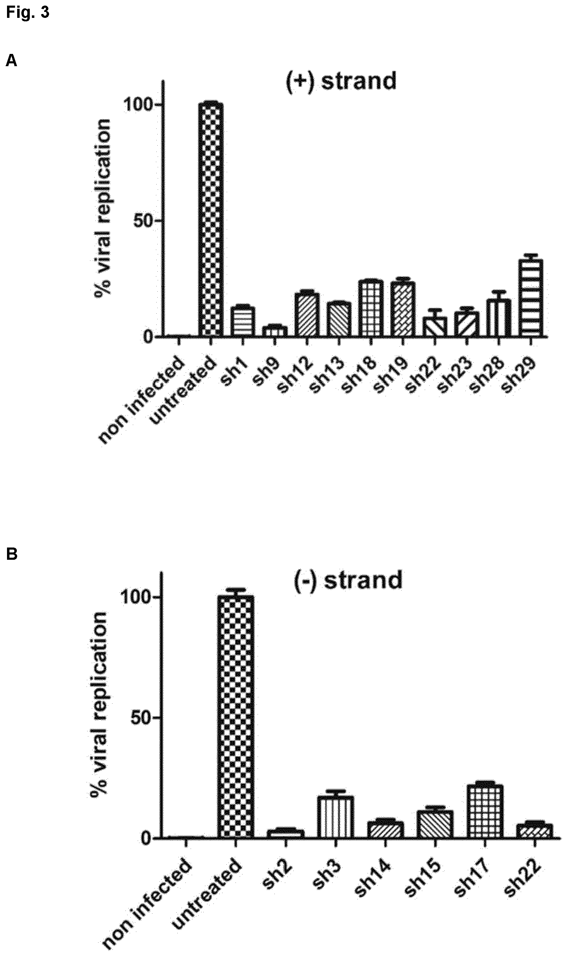

LLCMK2 cells were transduced lentivirally with 16 most effective shmiR30-cadidates (Table 8) three days prior to infection with a multiplicity of infection (MOI) of 1 for 6 hours. At the day of infection, cells were infected with HPIV3 (strain C 243, 2*10² focus forming units (FFU) in 200 µL). One hour post infection, the HPIV3 supernatant was withdrawn and cells were cultured for 40 hours at 35°C. Afterwards, supernatants were collected and HPIV3 titers determined by using a focus forming assay (FFA) on LLCMK2 cells (results in Fig. 3).

**Table 8: target sequences of candidates used for testing in LLCMK2 cells**

| **(+) strand** | | **(-) strand** | |
|---|---|---|---|
| **Name** | **Candidate Sequence** | **Name** | **Candidate Sequence** |
| LGene_195_v2 | SEQ ID NO: 1 | LGene_Neg_869_v2 | SEQ ID NO: 32 |
| LGene_952_v2 | SEQ ID NO: 9 | LGene_Neg_3739_v2 | SEQ ID NO: 33 |
| HNGene_1243_v2 | SEQ ID NO: 12 | HNGene_Neg_1440_v2 | SEQ ID NO: 44 |
| HNGene_903_v2 | SEQ ID NO: 13 | HNGene_Neg_1437_v2 | SEQ ID NO: 45 |
| FGene_1618_v2 | SEQ ID NO: 18 | FGene_Neg_71_v2 | SEQ ID NO: 47 |
| FGene_1595_v2 | SEQ ID NO: 19 | PCGene_Neg_1728_v2 | SEQ ID NO: 52 |
| PCGene_1787_v2 | SEQ ID NO: 22 | | |
| PCGene_1268_v2 | SEQ ID NO: 23 | | |
| MGene_592_v2 | SEQ ID NO: 28 | | |
| MGene_550_v2 | SEQ ID NO: 29 | | |

### siRNA candidate screening in an HPIV3 infection model in LLCMK2

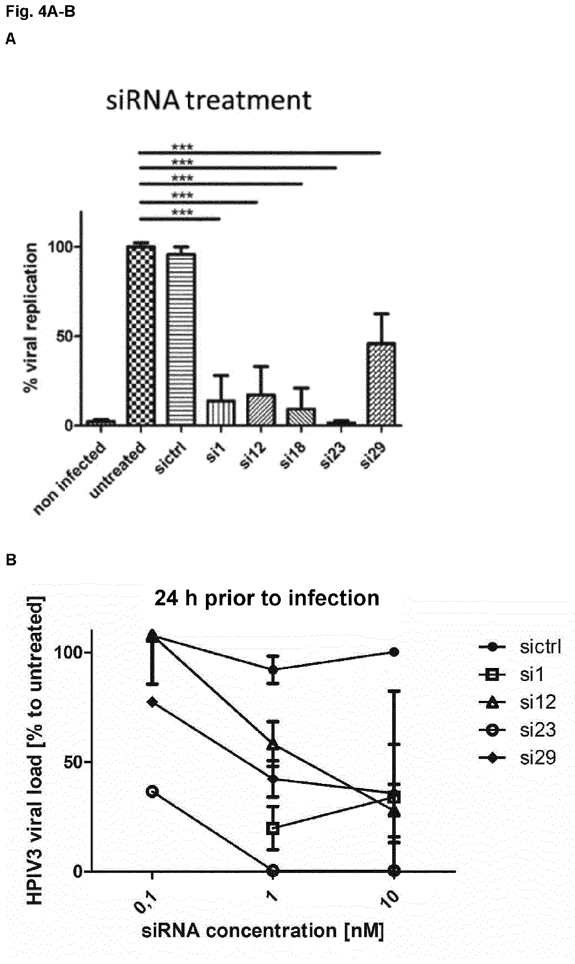

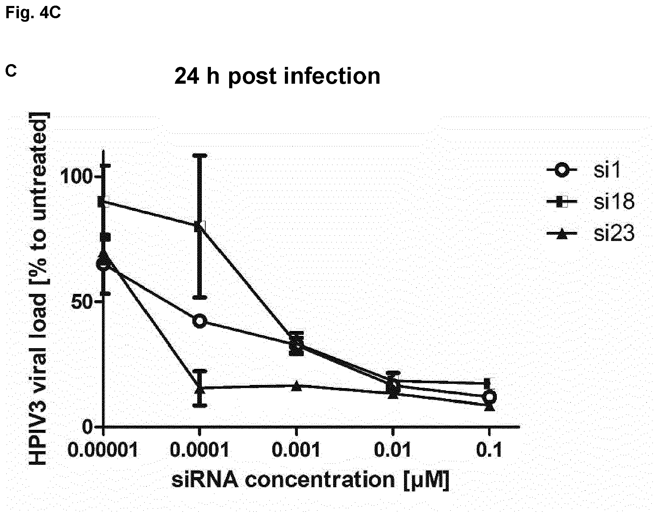

LLCMK2 cells were transfected with the siRNAs using Lipofectamine RNAi Max 24 hours prior to or 24 hours post infection with concentrations ranging from 0.01 nM to 0.1 µM. At the day of infection, cells were infected with HPIV3 (strain C 243, 2*10² focus forming units (FFU) in 200 µL). One hour post infection, the HPIV3 supernatant was withdrawn and cells were cultured for 40 hours at 35°C. Afterwards, supernatants were collected and HPIV3 titers determined by using a focus forming assay (FFA) on LLCMK2 cells (results in Fig. 4).

### Preparation of human precision-cut lung slices

Precision-cut lung slices (PCLS) were prepared as described elsewhere (Danov O et al., 2018 doi: 10.1371/journal.pone.0207767). Briefly, human explant lung tissue was filled with agarose, punched and cut with a tissue slicer to 200 - 300 µm thick round 8mm slices in diameter.

### shmiR30a candidate screening in an HPIV3 infection model in human precision-cut lung slices

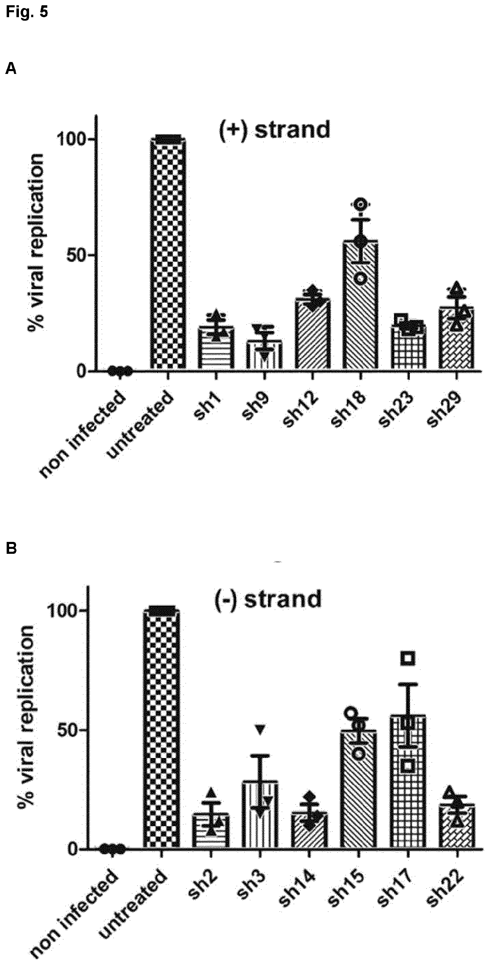

PCLS cells were transduced lentivirally with 12 most effective shmiR30-cadidates three days prior to infection with a multiplicity of infection (MOI) of 10 for 6 hours. At the day of infection, PCLS were infected with HPIV3 (strain C 243, 2*10⁴ FFU in 200 µL). One hour post infection, the HPIV3 supernatant was withdrawn and PCLS were cultured for 72 hours at 35°C. Afterwards, supernatants were collected and HPIV3 titers determined by using an FFA on LLCMK2 cells (results in Fig. 5).

### siRNA candidate screening in an HPIV3 infection model in human precision-cut lung slices

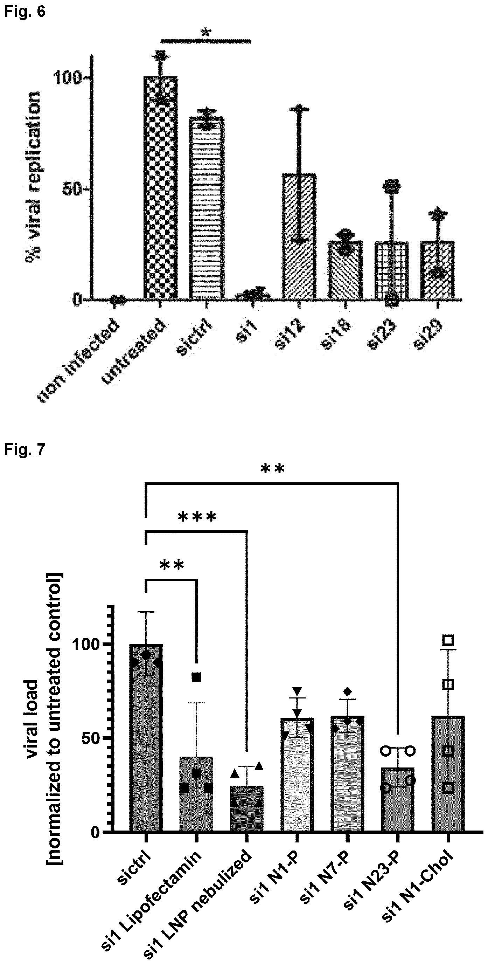

PCLS were transfected with the siRNAs using Lipofectamine RNAi Max 24 hours prior to infection with concentrations 1 µM. At the day of infection, cells were infected with HPIV3 (strain C 243, 2*10⁴ FFU in 200 µL). One hour post infection, the HPIV3 supernatant was withdrawn and PCLS were cultured for 72 hours at 35°C. Afterwards, supernatants were collected and HPIV3 titers determined by using a focus forming assay (FFA) on LLCMK2 cells (results in Fig. 6).

### Example 2

### Efficiency of siRNA candidates in an HPIV3 infection model in LLCMK2 cells using lipofectamine, a LNP formulation or directly palmityl (P) - or cholersterol (C) - modified siRNA

20.000 LLCMK2 cells were infected with 2*10² FFU HPIV3 and treated with the respectively modified siRNAs (comprising a sequence of SEQ ID NO: 169) 24 hours post infection without further formulation. Viral load was assessed 24 hours post treatment and compared to nonmodified siRNA delivered by Lipofectamine or LNP (Fig. 7). N1, N7 and N23 indicate the nucleotide position within the siRNA to which the palmityl (P) or cholesterol (C) modification was added.

### Example 3

### Pseudotyping of lentiviral particles with different pseudotype chymeras

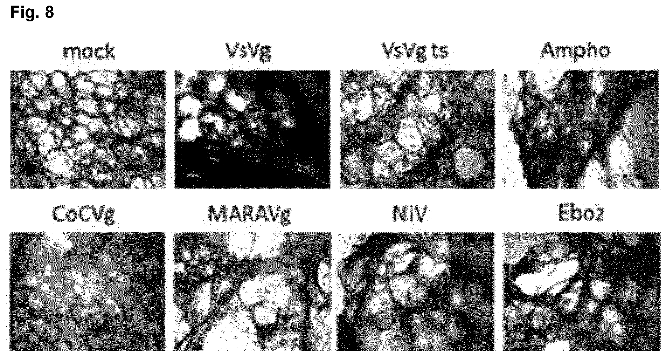

Initially, different lentiviral particles displaying vesiculoviral glycoprotein pseudotypes were screened for their transduction efficacy on PCLS (Fig. 8) On this basis, pseudotypes VSVg, MaraVg, CoCVg and PyriVg were determined to serve as a basis for further pseudotype chimeras to be generated.

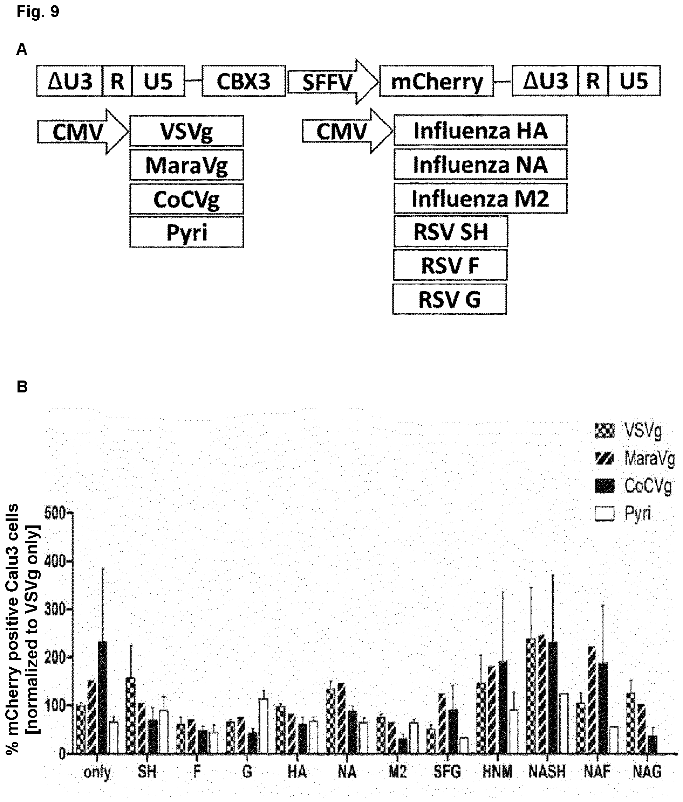

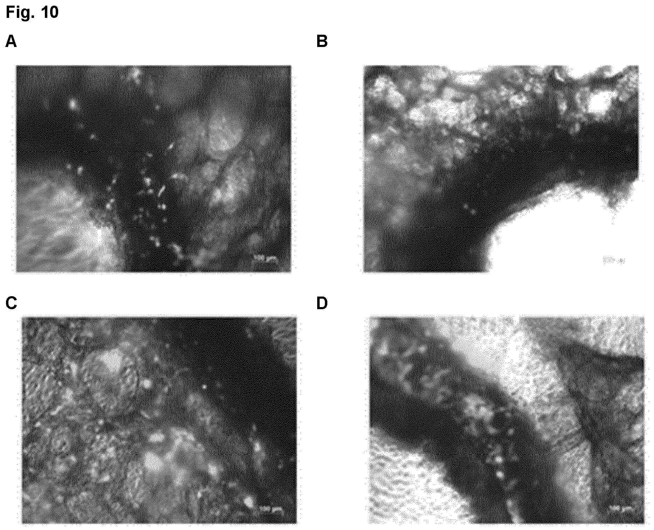

For this, RSV surface proteins F, G and SH as well as Influenza A surface proteins HA, NA and M2 were incorporated into the lentiviral particle surface upon vector production in various combinations (table 9). Differentially pseudotyped vectors were screened in Calu3 lung cells for their efficacy. For this, transduction was performed using an MOI of 1 for all candidates. 48 hours post transduction, cells were analyzed for mCherry expression by flow cytometry (schematic pseudotypes and results in Fig. 9). The most successful combinations VSVg/Influenza-NA/RSV-SH and MaraVg/Influenza-NA/RSV-SH were further tested in PCLS with MOls of 1 and 10, displaying a very high transduction efficiency (Fig. 10).

**Table 9: Exemplary combinations of different pseudotypes for construction of lentiviral chimeras**

| **Vesiculoviral glykoprotein** | **Combination partner** |
|---|---|
| VSVg | lnfl.-HA/lnfl.-NA/lnfl.-M2; InfI.SH/RSV-NA; Infl.-NA/RSV-F; Infl.-HA/Infl.-NA/RSV-F/RSV-SH; RSV-F/RSV-G/RSV-SH |
| MaraVg | lnfl.-HA/lnfl.-NA/lnfl.-M2; InfI.SH/RSV-NA; Infl.-NA/RSV-F; Infl.-HA/Infl.-NA/RSV-F/RSV-SH; RSV-F/RSV-G/RSV-SH |
| CoCVg | lnfl.-HA/lnfl.-NA/lnfl.-M2; InfI.SH/RSV-NA; Infl.-NA/RSV-F; Infl.-HA/Infl.-NA/RSV-F/RSV-SH; RSV-F/RSV-G/RSV-SH |
| Pyri-glycoprotein | lnfl.-HA/lnfl.-NA/lnfl.-M2; InfI.SH/RSV-NA; Infl.-NA/RSV-F; Infl.-HA/Infl.-NA/RSV-F/RSV-SH; RSV-F/RSV-G/RSV-SH |

### References:

Boncristiani HF, Criado MF, Arruda E. Respiratory Viruses. Encyclopedia of Microbiology. 2009:500-18. doi: 10.1016/B978-012373944-5.00314-X. Epub 2009 Feb 17. PMCID: PMC7149556.
Zhu, C., Lee, J.Y., Woo, J.Z. et al. An intranasal ASO therapeutic targeting SARS-CoV-2. Nat Commun 13, 4503 (2022).
Catalanotto, C., Cogoni, C., & Zardo, G., 2016, MicroRNA in Control of Gene Expression: An Overview of Nuclear Functions. International journal of molecular sciences, 17(10), 1712.
Lalle E, Biava M, Nicastri E, Colavita F, Di Caro A, Vairo F, Lanini S, Castilletti C, Langer M, Zumla A, Kobinger G, Capobianchi MR, Ippolito G. Pulmonary Involvement during the Ebola Virus Disease. Viruses. 2019 Aug 24;11(9):780. doi: 10.3390/v11090780. PMID: 31450596; PMCID: PMC6784166.
Devnath P, Masud HMAA. Nipah virus: a potential pandemic agent in the context of the current severe acute respiratory syndrome coronavirus 2 pandemic. New Microbes New Infect. 2021 May;41:100873. doi: 10.1016/j.nmni.2021.100873. Epub 2021 Mar 19. PMID: 33758670; PMCID: PMC7972828.
Pelossof, Fairchild, et al. "Prediction of potent shRNAs with a sequential classification algorithm." Nature Biotechnology. 2017 Apr;35(4):350-353.
Adams FF, Heckl D, Hoffmann T, Talbot SR, Kloos A, Thol F, Heuser M, Zuber J, Schambach A, Schwarzer A. An optimized lentiviral vector system for conditional RNAi and efficient cloning of microRNA embedded short hairpin RNA libraries. Biomaterials. 2017 Sep;139:102-115. doi: 10.1016/j.biomaterials.2017.05.032. Epub 2017 May 23. PMID: 28599149.
Fellmann C, Zuber J, McJunkin K, Chang K, Malone CD, Dickins RA, Xu Q, Hengartner MO, Elledge SJ, Hannon GJ, Lowe SW. Functional identification of optimized RNAi triggers using a massively parallel sensor assay. Mol Cell. 2011 Mar 18;41(6):733-46. doi: 10.1016/j.molcel.2011.02.008. Epub 2011 Feb 25. PMID: 21353615; PMCID: PMC3130540.
Selvam C, Mutisya D, Prakash S, Ranganna K, Thilagavathi R. Therapeutic potential of chemically modified siRNA: Recent trends. Chem Biol Drug Des. 2017 Nov;90(5):665-678. doi: 10.1111/cbdd.12993. Epub 2017 May 16. PMID: 28378934; PMCID: PMC5935465. https://en.wikipedia.org/wiki/Solid_lipid_nanoparticle
Gan SW, Tan E, Lin X, Yu D, Wang J, Tan GM, Vararattanavech A, Yeo CY, Soon CH, Soong TW, Pervushin K, Torres J. The small hydrophobic protein of the human respiratory syncytial virus forms pentameric ion channels. J Biol Chem. 2012 Jul 13;287(29):24671-89. doi: 10.1074/jbc.M111.332791. Epub 2012 May 23. PMID: 22621926; PMCID: PMC3397895.
Johnson SM, McNally BA, loannidis I, Flano E, Teng MN, Oomens AG, Walsh EE, Peeples ME. Respiratory Syncytial Virus Uses CX3CR1 as a Receptor on Primary Human Airway Epithelial Cultures. PLoS Pathog. 2015 Dec 11;11(12):e1005318. doi: 10.1371/journal.ppat.1005318. PMID: 26658574; PMCID: PMC4676609.
Trobridge et al., 2010, Cocal-pseudotyped Lentiviral Vectors Resist Inactivation by Human Serum and Efficiently Transduce Primate Hematopoietic Repopulating Cells. Molecular Therapy 18(4), 725-733.
German Advisory Committee Blood (Arbeitskreis Blut), Subgroup 'Assessment of Pathogens Transmissible by Blood', 2016, Human Immunodeficiency Virus (HIV). Transfus Med Hemother. 43(3), 203-222
Naldini et al., 1996, In vivo gene delivery and stable transduction of nondividing cells by a lentiviral vector. Science 272(5259). 263-267.
Zufferey et al., 1998, Self-Inactivating Lentivirus Vector for Safe and Efficient in Vivo Gene Delivery. J Virol 72(12), 9873-9880.
Schambach et al., 2013, Biosafety Features of Lentiviral Vectors. Human Gene Therapy 24, 132-142.
Shaw and Cornetta, 2014, Design and Potential of Non-Integrating Lentiviral Vectors. Biomedicines 2(1), 14-35.
Tijani et al., 2018, Lentivector Producer Cell Lines with Stably Expressed Vesiculovirus Envelopes. Mol Ther Methods Clin Dev. 10, 303-312.
Brown KM, Nair JK, Janas MM, Anglero-Rodriguez YI, Dang LTH, Peng H, Theile CS, Castellanos-Rizaldos E, Brown C, Foster D, Kurz J, Allen J, Maganti R, Li J, Matsuda S, Stricos M, Chickering T, Jung M, Wassarman K, Rollins J, Woods L, Kelin A, Guenther DC, Mobley MW, Petrulis J, McDougall R, Racie T, Bombardier J, Cha D, Agarwal S, Johnson L, Jiang Y, Lentini S, Gilbert J, Nguyen T, Chigas S, LeBlanc S, Poreci U, Kasper A, Rogers AB, Chong S, Davis W, Sutherland JE, Castoreno A, Milstein S, Schlegel MK, Zlatev I, Charisse K, Keating M, Manoharan M, Fitzgerald K, Wu JT, Maier MA, Jadhav V. Expanding RNAi therapeutics to extrahepatic tissues with lipophilic conjugates. Nat Biotechnol. 2022 Oct;40(10):1500-1508. doi: 10.1038/s41587-022-01334-x. Epub 2022 Jun 2. PMID: 35654979.
Raouane M, Desmaële D, Urbinati G, Massaad-Massade L, Couvreur P. Lipid conjugated oligonucleotides: a useful strategy for delivery. Bioconjug Chem. 2012 Jun 20;23(6):1091-104. doi: 10.1021/bc200422w. Epub 2012 Mar 15. PMID: 22372953.
Schwarz and Merkel, 2017, Functionalized PEI and Its Role in Gene Therapy. Material Matters, 2017, 12.2 available at: https://www.sigmaaldrich.com/DE/de/technical-documents/technical-article/materials-science-and-engineering/drug-delivery/functionalized-pei- and-its-role-in-gene-therapy.
Du Q, Thonberg H, Wang J, Wahlestedt C, Liang Z. A systematic analysis of the silencing effects of an active siRNA at all single-nucleotide mismatched target sites. Nucleic Acids Res. 2005;33:1671-1677.
Ahmed F, Raghava GP. Designing of highly effective complementary and mismatch siRNAs for silencing a gene. PLoS One. 2011;6(8):e23443.
Liu B, Brendel C, Vinjamur DS, Zhou Y, Harris C, McGuinness M, Manis JP, Bauer DE, Xu H, Williams DA. Development of a double shmiR lentivirus effectively targeting both BCL11A and ZNF410 for enhanced induction of fetal hemoglobin to treat β-hemoglobinopathies. Mol Ther. 2022 Aug 3;30(8):2693-2708. doi: 10.1016/j.ymthe.2022.05.002. Epub 2022 May 6. PMID: 35526095; PMCID: PMC9372373.

## Claims

1. An oligonucleotide molecule capable of targeting an RNA derived from human parainfluenza virus, wherein the oligonucleotide molecule is
a) an RNA molecule capable of inducing RNAi comprising a nucleic acid sequence having at least 90% sequence identity to any of SEQ ID NO: 1 to 56, preferably to SEQ ID NO: 1, 18 or 23; or
b) an antisense oligonucleotide comprising a nucleic acid sequence that corresponds at least to a subsequence of 10 or more nucleotides of any of SEQ ID NO: 1 to 56, preferably of SEQ ID NO: 1, 8 or 23.

2. The oligonucleotide molecule of claim 1, wherein the oligonucleotide is an RNA molecule comprising a nucleic acid sequence of SEQ ID NO: 1 or an antisense oligonucleotide comprising a nucleic acid sequence of SEQ ID NO: 204.

3. The oligonucleotide molecule of claim 1, wherein the oligonucleotide is an RNA molecule comprising a nucleic acid sequence of SEQ ID NO: 18 or an antisense oligonucleotide comprising a nucleic acid sequence of SEQ ID NO: 205.

4. The oligonucleotide molecule of claim 1, wherein the oligonucleotide is an RNA molecule comprising a nucleic acid sequence of SEQ ID NO: 23 or an antisense oligonucleotide comprising a nucleic acid sequence of SEQ ID NO: 206.

5. A screening method for an oligonucleotide-based therapeutic for treating an infection with a respiratory virus of interest, the method comprising:
a) identifying at least one target sequence in an RNA derived from the respiratory virus of interest;
b) generating one or more candidate oligonucleotide molecules comprising a nucleic acid sequence complementary to the at least one target sequence identified in step a), wherein the candidate oligonucleotide molecule is selected from the group consisting of an RNA molecule capable of inducing RNAi and an antisense oligonucleotide;
c) screening the candidate oligonucleotide molecules generated in step b) for their ability to induce a reduction of the titer of the respiratory virus of interest in lung tissue wherein the screening comprises steps of
i. providing cut slices obtained from lung tissue
ii. introducing the one or more candidate oligonucleotide molecules generated in step b) into the cut slices,
iii. infecting the cut slices with the respiratory virus of interest,
iv. obtaining supernatant from the cut slices at a first time point and at least a second time point after infection;
v. determining the respiratory virus titre in the supernatants obtained at the first time point and at the at least second time point of step iv. for each candidate oligonucleotide molecule,
wherein steps c) ii. and iii. may be performed in any order.

6. The method of claim 5, further comprising at least one validation step prior to step c) comprising:
i. cloning an RNA sequence identified in step a) or parts thereof into an UTR of an mRNA molecule encoding a fluorescent protein to obtain a reporter mRNA;
ii. introducing said reporter mRNA into a cell,
iii. introducing the one or more candidate oligonucleotide molecules generated in step b) into the cell,
iv. Assessing the fluorescence intensity in the cells comprising the reporter mRNA to determine a knock-down efficiency for each candidate oligonucleotide molecule,
wherein, preferably, those candidate oligonucleotide molecules having a knock-down efficiency of about 70%, preferably, 80%, most preferably 90% are subjected to the screening of step c) of claim 5; and/or
I. introducing the one or more oligonucleotide RNA molecules generated in step b) into a cell in cell culture,
II. infecting the cell of (I) with the respiratory virus of interest,
III. obtaining supernatant from the cell of (I) at a first time point and at least a second time point after infection;
IV. Determining the virus titre in the supernatants obtained at the first time point and at the at least second time point of step (III),
wherein, preferably, an oligonucleotide molecule is subjected to the screening of step c) of claim 5 when the viral titre in the supernatant of the second time point is reduced by more than 50%, preferably by more than 60%, more preferably by more than 70%, most preferably more than 80% compared to the viral titre in the supernatant of the first time point.

7. A method of preparing an oligonucleotide-based therapeutic for treating an infection with a respiratory virus of interest, the method comprising steps of selecting a candidate oligonucleotide molecule capable of targeting an RNA derived from a respiratory virus using the screening method of any of claims 5 or 6 and providing a vector comprising or encoding said candidate oligonucleotide molecule to obtain the oligonucleotide-based therapeutic for treating an infection with a respiratory virus of interest, wherein, optionally, the oligonucleotide-based therapeutic is formulated in a pharmaceutical composition.

8. The screening method of any of claims 5 or 6 or the method of preparing an oligonucleotide-based therapeutic of claim 7, wherein the respiratory virus of interest is selected from the group comprising influenza virus, respiratory syncytial virus, parainfluenza virus, metapneumovirus, rhinovirus, coronavirus, adenovirus, bocavirus, Ebola virus and Nipah virus.

9. An oligonucleotide-based therapeutic for treating an infection with a human parainfluenza virus, wherein the therapeutic comprises one or more of the oligonucleotide molecules of any of claims 1 to 4 or, if the oligonucleotide molecule is an RNA molecule capable of inducing RNAi, a nucleic acid encoding them, preferably
a) a lentiviral vector comprising a nucleic acid encoding one or more of the RNA molecules of any of claims 1 to 4,
b) a lipid nanoparticle comprising one or more of the oligonucleotide molecules of any of claims 1 to 4,
c) a cationic polymer selected from the group comprising a PEI vector associated with one or more of the oligonucleotide molecules of any of claims 1 to 4, or
d) one or more of the oligonucleotide molecules of any of claims 1 to 4 linked to a lipophilic moiety, preferably 2'-O-hexadecyl.

10. The oligonucleotide molecule of any of claims 1 to 4, the screening method of any of claims 5, 6 or 8, the method of preparing an oligonucleotide-based therapeutic of any of claims 7 or 8 or the oligonucleotide-based therapeutic of claim 9, wherein the oligonucleotide molecule is an RNA molecule capable of inducing RNAi selected from the group comprising small interfering RNAs, short hairpin RNAs and artificial microRNAs.

11. The oligonucleotide molecule of any of claims 1 to 4, the screening method of any of claims 5, 6 or 8, the method of preparing an oligonucleotide-based therapeutic of any of claims 7 or 8 or the oligonucleotide-based therapeutic of claim 9, wherein the oligonucleotide molecule is an antisense oligonucleotide selected from the group comprising a DNA antisense oligonucleotide and a gapmer.

12. A composition for use in treating a respiratory disease in a subject, wherein said composition is administered to the airways of the subject, wherein the composition comprises a lentiviral vector pseudotyped with a vesiculoviral envelope glycoprotein and at least one surface protein of influenza A virus and/or at least one surface protein of respiratory syncytial virus, preferably, with Influenza NA.

13. The composition fur use of claim 12, wherein the lentiviral vector is pseudotyped with a vesiculoviral envelope glycoprotein, at least one surface protein of respiratory syncytial virus and at least one surface protein of influenza A virus.

14. The composition for use of any of claims 12 or 13, wherein the vesiculoviral envelope glycoprotein is selected from the group comprising MaraVg, VSVg, CoCVg and PyriVg, preferably, MaraVg or VSVg.

15. The composition for use of any of claims 12 to 14, wherein the at least one surface protein of respiratory syncytial virus is selected from the group comprising surface proteins SH, F and G, preferably, SH.

16. The composition for use of any of claims 12 to 15, wherein the at least one surface protein of influenza A virus is selected from the group comprising surface proteins NA, HA and M2, preferably, NA.

17. The composition for use of any of claims 12 to 16, further comprising a nucleic acid encoding one or more of the RNA molecules of any of claims 1 to 4.
